# EUROPEAN PATENT APPLICATION

(11) **EP 1 992 360 A1**
(43) Date of publication of application: **19.11.2008**
(21) Application number: 06797491.5
(22) Date of filing: 30.08.2006
(51) Int. Cl.: A61K 45/00, A61K 31/4439, A61K 31/4709, A61K 31/5025, A61K 31/506, A61K 31/7088, A61K 38/00, A61P 35/00, A61P 43/00

(54) **JOINT USE OF TGF-BETA SIGNAL INHIBITOR AND ANTITUMOR AGENT**

(30) Priority: 01.02.2006 JP 2006024843; 01.02.2006 JP 2006024845
(71) Applicant: The University of Tokyo, Bunkyo-ku, Tokyo 113-8654 (JP)
(72) Inventor: KATAOKA, Kazunori, Tokyo 1650031 (JP); MIYAZONO, Kohei, Shiki-shi, Saitama 3530004 (JP); KANO, Mitsunobu, Tokyo 1350022 (JP); BAE, Younsoo, Tokyo 1200014 (JP); NISHIYAMA, Nobuhiro, Tokyo 1140002 (JP); HIRAKAWA, Kosei, Osaka-shi, Osaka 5450035 (JP); YASHIRO, Masakazu, Hirakata-shi, Osaka 5731134 (JP); NODE, Manabu, Hirakata-shi, Osaka 5731118 (JP)
(74) Representative: Albrecht, Thomas
(86) International application number: PCT/JP2006/317593
(87) International publication number: WO 2007/088651

(57) **Abstract**

The combined use of a TGF- β signaling inhibitor and an antitumor active substance or an imaging agent modified by a drug-encapsulating macromolecular micelle, or the like, is provided. The selective delivery capability of the antitumor active substance or the imaging agent to the target is improved, increasing the antitumoral activity in the target.

## Description

### Technical Field

The present invention relates to use of transforming growth factor β (TGF-β) signaling inhibitor, for increasing leakiness neovasculature in tumor (or cancer) tissue, and depending on the circumstances, for decreasing intratumoral fibrous components; in addition, it relates to combined use and combination of the inhibitor and an antitumor agent.

### Background Art

Drug-encapsulating macromolecular micelle compounds developed as drug delivery system (DDS) for delivering selectively a drug to solid cancer, for instance, drug-encapsulating macromolecular micelles that encapsulate adriamycin or cisplatin in a macromolecular micelle comprising a block copolymer of polyethyleneglycol and poly-aspartic acid or polyethyleneglycol and poly-glutamic acid, have been recognized to demonstrate higher efficacy against a given type of cancer compared to the corresponding non-modified drugs alone (for instance, refer to Patent Reference 1 or Patent Reference 2 respectively; hereinafter, the literature referred to in the related art will be given at the end of this section). Then, both parties are at the stage of clinical study, with forecasted practical uses. However, not only existing chemotherapeutic agents but also the above drug-encapsulating macromolecular micelles do not exert a sufficient therapeutic effect yet, for instance, in cancers having characteristics such as defective vascular architecture in cancer stroma (containing mainly fibrous components such as neovasculature and collagenous fiber) or thick fibrous components of the stroma, which remain difficult to treat.

Meanwhile, the role of TGF-β signaling is being studied intensively in relation to inhibition of growth or metastasis of cancers, where TGF-β signaling is known to be involved in angiogenesis, or the like. Of blood vessels in general, regulation of TGF-β signaling is known to be a method for regulating permeability (refer to Patent Reference 3). However, of angiogenesis, in particular of angiogenesis related to cancer, there is no established view yet on whether TGF-β alone is a promoting factor or an inhibitory factor (refer to Non-patent Reference 1). Concretely, a TGF-β ligand is secreted outside the cell in an inactive, latent form. From this latent TGF- β form, a matured form is further excised, which acquires activity and first binds to a Type II receptor (T β RII) on the target cell surface. The Type II receptor has high binding affinity for TGF-β. At this point of time, a Type III receptor, also called co-receptor, is capable of ligand binding although there is no intracellular signal transduction activity. The bound Type II receptor recruits a Type I receptor (in general ALK5 and ALK1 depending on the circumstances) to form a complex. This complex next activates the Smad pathway, which is a group of molecules in charge of intracellular communication mechanism. That is to say, first, Smad2/3 is phosphorylated (Smad1/5/8 if downstream from ALK1), this binds to Smad4 and translocates into the nucleus, leading to the initiation of translation of the target molecule inside the nucleus (refer to the above Non-patent Reference 1 and Non-patent Reference 2).

In addition, regarding the role of TGF-β signaling in general related to cancer, TGF- β is known to work for growth inhibition in normal epithelial, endothelial and hematopoietic cells (for instance, refer to Non-patent Reference 3, Non-patent Reference 4 and Non-patent Reference 5). That is to say, in a normal tissue or a TGF-β-responsive benign tumor tissue, TGF-β has carcinogenesis inhibition effect. On the other hand, it is thought that, when a cancer becomes malignant to some extent, often becoming non-responsive to growth inhibition by TGF-β, on the contrary, cancer cells *per se* produce TGF- β in large amounts, prompting growth of surrounding tissue (cancer stroma), which becomes a scaffold, and promoting the development and metastasis of the tumor. From the point of view that multiple diseases are related to such overproduction of TGF- B, a TGF- β signal transduction inhibitor has been provided, which is a pyrrolopyrazole derivative, as an agent to be applied to these diseases (refer to Patent Reference 4). In this reference, taking into consideration mechanism such as cancer cells *per se* produce TGF-β in large amounts, a suggestion is made, that the pyrrolopyrazole derivative alone or a combination with another antitumor agent is thought to be advantageous for the treatment of advanced cancer.

However, data, such as, against which cancer and at which level [the treatment] was effective, are not disclosed whatsoever, and regarding the concrete efficacy thereof, nothing has been identified whatsoever.

In addition, it has been suggested that, if a TGF-β signaling is inhibited in a cancer cell of a benign epithelial cancer, the carcinogenesis process is promoted (refer to Non-patent Reference 6, Non-patent Reference 7 and Non-patent Reference 8).

In addition, TGF-β has immuno-inhibitory effect (refer to Patent Reference 5). Therefore, TGF-β production by tumor cell is thought to attenuate immunity against tumor (refer to Non-patent Reference 9), then, tumors are known to disappear in thymoma and malignant melanoma models if T-cells become non-responsive to TGF- β (refer to Non-patent Reference 10).

As indicated above, role of TGF-β signaling is also described as being efficient from the point of view of cancer treatment, or on the contrary, sometimes having adverse effects.

In addition, a number of observations also exist regarding antitumoral activities of various substances involved in the TGF-β signal transduction pathway. For instance, it is known that lung metastasis decreased in a mouse syngeneic lung metastasis model of mouse breast cancer cell line 4T1 when solubilized TGF- β Type II receptor (TGF- β ligand-adsorbed) protein was systemically administered by intraperitoneal injection (refer to Non-patent Reference 11), and that in a chronic hepatitis model, when the above protein was administered systemically by intraperitoneal injection, hepatic fibrosis was suppressed at 5mg/kg, but fibrosis was not suppressed at 2.5mg/kg (refer to Non-patent Reference 12). In addition, a number of anti-TGF- β antibodies (ligand neutral) *per se* in stand-alone use mode are already in clinical trials in Europe and America, some of which having reached Phase III (refer to Non-patent Reference 13, Non-patent Reference 14). Similarly, some TGF-β antisense oligonucleotides (TGF- β gene transcription inhibition) are also in clinical trials (refer to Non-patent Reference 15).

In addition to the above high molecular weight compounds, research has been undertaken actively for low molecular weight TGF-β signaling inhibitors as well. For instance, LY364947 (TGF- β Type I receptor (at leastALK5) phosphorylation inhibitor by ATP-competitive inhibition) (refer to Non-patent Reference 16), A77-01 (phosphorylation inhibitor similar to LY364947) (refer to Non-patent Reference 17), and other various low molecular weight TGF-β signaling inhibitors are under development as antitumor agents (refer to the above Non-patent Reference 2).
[Patent Reference 1] Japanese Patent Publication No. 2517760
[Patent Reference 2] WO Publication No. 2002/26241
[Patent Reference 3] WO Publication No. 2005/013915
[Patent Reference 4] National Publication of Translated Version No. 2004-535404 (or WO Publication No. 2002/094833)
[Patent Reference 5] National Publication of Translated Version No. 2006-503899 (or WO Publication No. 2004/034023)
[Non-patent Reference 1] Mitsunobu Kano, Kohei Miyazono; The Roles of TGF-β Superfamily Signals During Vascularization, Molecular Medicine 2005; 42; 661-668, Japanese Review Article
[Non-patent Reference 2] Yingling, J. M. et al., Nature Rev. Drug Disc. 3, 1011-1022 (2004)
[Non-patent Reference 3] Siegel, P. M. et al., Nature Rev. Cancer 3, 807-818 (2003)
[Non-patent Reference 4] Wakefield, I. M. et al., Curr. Opin. Gen. Dev. 12, 22-29 (2002)
[Non-patent Reference 5] Dumont, N, et al., Cancer Cell 3, 531-536 (2003)
[Non-patent Reference 6] Tang, B. et al., J. Clin. Invest. 112, 1116-1124 (2003)
[Non-patent Reference 7] Cui, W. et al., Cell 86, 531-542 (1996)
[Non-patent Reference 8] Oft, M. et al., Nature Cell Biol. 4, 487-494 (2002)
[Non-patent Reference 9] Letterio, J. J. et al., Annu. Rev. Immunol. 16, 137-161 (1998)
[Non-patent Reference 10] Gorelik, I. et al., Nature Med. 7, 1118-1122 (2001)
[Non-patent Reference 11] Muraoka, R. et al., J. Clin. Invest. 109, 1551-1559 (2002)
[Non-patent Reference 12] George, J. et al., Proc. Natl. Acad. Sci. USA 96, 12719-12724 (1999)
[Non-patent Reference 13] Mead, A. I. et al., Invest. Ophthalmol. Vis. Sci. 44, 3394-3401 (2003)
[Non-patent Reference 14] Mealy, N. E. et al., Drugs. Fut. 28, 320-322 (2003)
[Non-patent Reference 15] Bogdahn, U. et al., Am. Soc. Clin. Oncol. Ann. Meet. Abstract 1514 (2004)
[Non-patent Reference 16] Sawyer, J. S. et al., J. Med. Chem. 46, 3953-3956 (2003)
[Non-patent Reference 17] Tojo, M. et al., Cancer Sci. 96: 791-800 (2005)

As described above, with drug-encapsulating macromolecular micelles, improvement in delivery capability to target cell or tissue has been confirmed, and a given satisfactory efficacy has been obtained; in addition, based on totally different observations from prior art observations, antitumor agents falling in a novel category are being proposed. However, from the point of view of using antitumor agents with generally high toxicity, provision of medicinal formulation allowing for further improvement of target selectivity and accessibility and lower doses will be desired. In particular, provision of a medicinal formulation demonstrating a significant effect in cancers with a defective vascular architecture or a strong fibrosis tissue type (pancreatic adenocarcinoma, scirrhous gastric cancer and the like) is desired.

The present inventors found that, when LY364947, a low molecular weight TGF-β Type I receptor, was administered at low doses that did not allow access to cancer cells *per se,* or, when solubilized TGF-β Type II receptor protein was administered at a low dose described as not inhibiting hepatic fibrosis in a chronic hepatitis model, for instance, in the above Non-patent Reference 12, the leakiness of neovasculature was significantly increased in the tumor xenograft of a tumor xenograft model in which Smad4-deficient mutant tumor cell line was transplanted in a nude mouse, compared to non-treated ones. In addition, they also found that, on the other hand, no alteration of leakiness was exerted on existing vasculature of general organs.

In addition, the present inventors observed that, when such an enhancement of leakiness is exerted on the neovasculature, delivery of an antitumor agent or a imaging agent selectively to a tumor cell became facilitated; in addition, if an antitumor agent, in particular, a modified antitumor agent, which delivery capability to tumor cell or tumor tissue has been improved by modifying an antitumor active substance, and a TGF-β signaling inhibitor are used in combination, actually, the therapeutic effect against pancreatic adenocarcinoma, scirrhous gastric cancer metastatic foci, and the like, was significantly increased. This effect means that an extremely promising cancer chemotherapy can be provided, taking into consideration that, although a combination therapy with an anti-VEGF antibody (Bevacizumab; trade name Avastin) and a general anticancer agent (refer to MacKenzie, M.J. et al., Lancet Oncol 5: 541-49 (2004) and de Gramont, A. et al., Oncology. 2005; 69 Suppl 3: 46-56.) already approved in the U.S. demonstrated a remarkable effect also clinically in a number of cancers (in particular, metastatic large bowel cancer), a significant combined application effect is not clear on pancreatic adenocarcinoma. In addition to this, if the dose of the TGF-β signaling inhibitor administered is increased gradually, for instance, as reported also in the above Non-patent Reference 12, fibrosis can be inhibited further, allowing for an improvement or an enhancement of drug delivery capability for other drugs through inhibition of fibrosis, without stopping at adjustment of leakiness of neovasculature.

### Disclosure of the Invention

Therefore, according to the present invention, a medicinal formulation is provided, containing a TGF- β signaling inhibitor as an active ingredient, increasing the leakiness of neovasculature in a tumor tissue, and, depending on the circumstances, additionally decreasing fibrosis.

In addition, as another mode of this usage mode, use of TGF-β signaling inhibitor to prepare a medicinal formulation for rendering a neovasculature in a tumor tissue leaky is provided, and furthermore, a method is also provided, to improve the efficacy of antitumor active substance in an individual, comprising administering an effective dose of TGF-β signaling inhibitor to an individual (a mammal and, in particular, a human) requiring the neovasculature in a tumor tissue to become leaky.

As an invention of another mode, a combination for tumor treatment is provided, combining a TGF-β signaling inhibitor as an active ingredient and an antitumor active substance, which is an antitumor active substance as an active ingredient, modified so as to improve delivery capability to a tumor cell or a tumor tissue.

As an invention of a further other mode, a tumor selective imaging method and a combination for imaging are provided, combining a TGF-β signaling inhibitor and an imaging agent, preferably, an imaging agent modified so as to improve delivery capability to a tumor cell or a tumor tissue.

In the present specification, the term antitumor active substance, antitumor agent or anticancer agent is used interchangeably, and indicates, regardless of the type, mechanism of action and the like, of the compound, a substance having some sort of efficacy against a tumor, in particular a malignant tumor, of a mammal, in particular, a human. TGF-β and TGF- β signaling inhibitor are used with the content of the meaning generally recognized in the relevant technical field, used in the above Non-patent Reference 1 or the like. Briefly, TGF- β signaling inhibitor (or also called TGF- β signal transduction inhibitor) can be defined as a substance that inhibits the TGF- β signal transduction system path by inhibiting any of the factors constituting the TGF- β signal transduction system path, that is to say, TGF- β ligand, TGF- β Type I receptors (ALK5 and 1), TGF- β Type II receptor, TGF- β Type III receptors (beta-glycan and endoglin), Smad proteins (1, 2, 3, 4, 5 and 8) or a combination thereof.

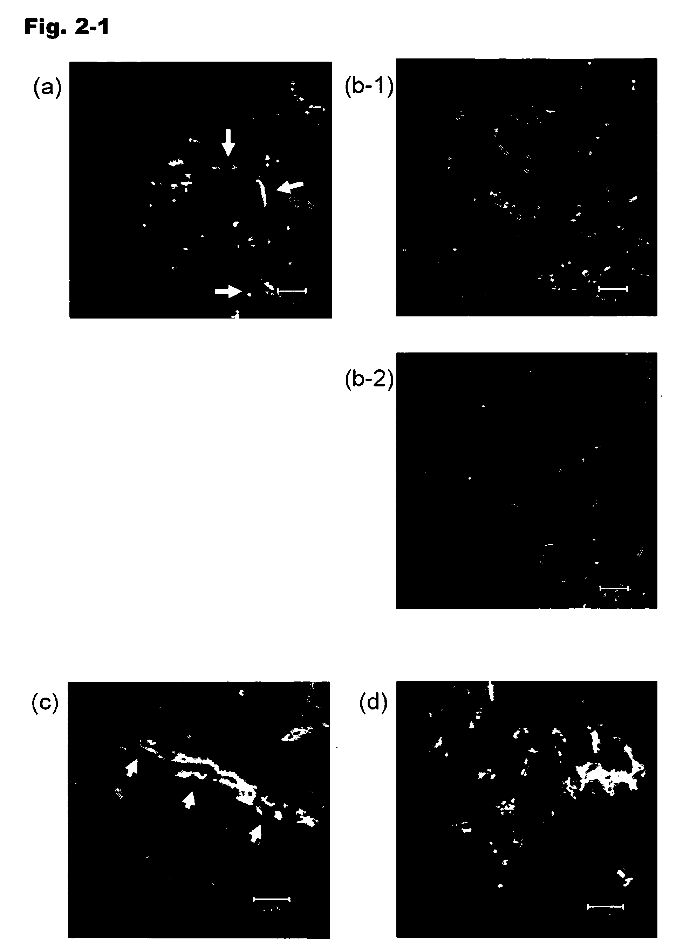

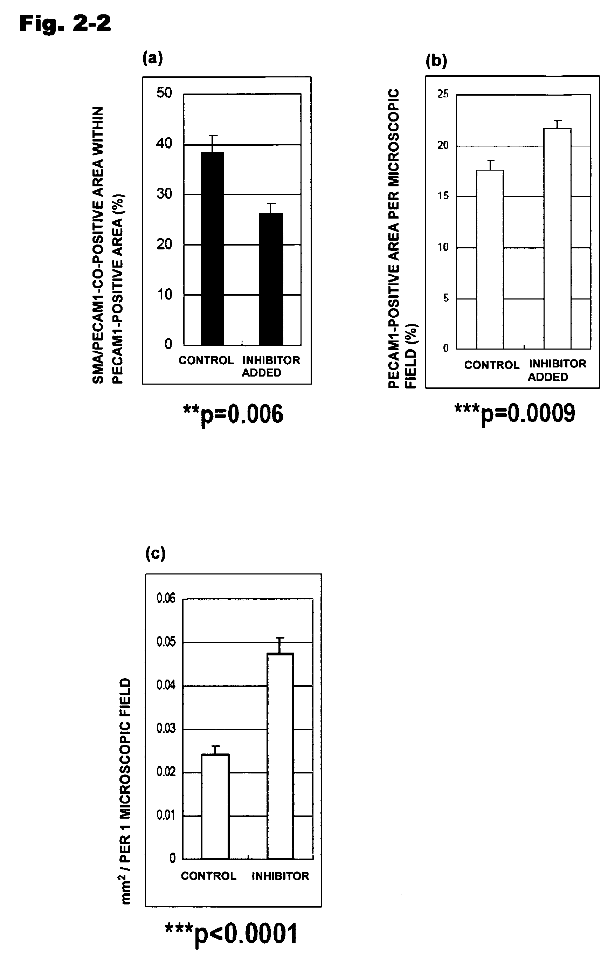

In the present invention, increasing the leakiness of neovasculature in tumor tissue, and, depending on the circumstances, decreasing fibrosis, are phenomena that can be observed, for instance, in a dextran injection experiment (briefly, an experiment in which a mouse is used, a Matrigel in which VEGF-A and FGF-2 have been mixed is injected subcutaneously into the abdominal wall, and seven days after administering TGF- β signaling inhibitor intraperitoneally, 2,000,000 molecular weight dextran is injected, the animal is sacrificed, and the relationship between neovasculature and dextran distribution in the extracted Matrigel plug is determined: refer to Kano, M.R.et al., J. Cell Sci.,118,3759-3768 (2005)) (refer to Fig. 2-1 and Fig. 2-2).

According to theory, although not restrictive, the increase in the leakiness of the neovasculature in a tumor tissue, then, depending on the circumstances, the decrease in fibrous component in the tumor, are thought to improve the delivery capability of antitumor active substances or antitumor agents, in particular, antitumor agents transformed into macromolecules, as well as antitumor agents modified by being encapsulated into liposomes, macromolecular micelles and the like, in particular, nanospheres, or the like, having sizes from several nm to several hundreds of nm in average diameter, or similarly modified imaging agents, to cancer cells.

The TGF- β signaling inhibitor usable in the present invention is conceptually included in the above definition, and, regardless of the type and source of the compound, any one is included as long as it is one that suits the object of the present invention (for instance, allows the leakiness in the neovasculature in tumor tissue to be increased, then, depending on the circumstances, the fibrous component in tumor to be decreased).

Although not limiting, for instance, high molecular weight substances, such as, soluble TGF-β Type I receptor, soluble TGF-β Type II receptor, soluble TGF- β Type III receptor, anti-TGF- β antibody, anti-TGF-β Type I receptor antibody, anti-TGF-β Type II receptor antibody and anti-TGF- β Type III receptor antibody, TGF-β antisense oligonucleotide or siRNA can be cited.

In addition, low molecular weight compounds having TGF-β Type I receptor kinase inhibitor activity are included in TGF- β signaling inhibitors, and although not limiting, for instance, dihydropyrrolopyrazole-based scaffold, imidazole-based scaffold, pyrazolopyridine-based scaffold, pyrazole-based scaffold, imidazopyridine-based scaffold, triazole-based scaffold, pyridopyrimidine-based scaffold, pyrrolopyrazole-based scaffold and isothiazole-based scaffold can be cited. Such-and-such-based scaffold referred to here can also be referred to as a compound having such-and-such as the basic backbone. As typical such low molecular compounds, for instance, scaffolds comprising as the main body the structures represented by the following chemical formulae can be cited.

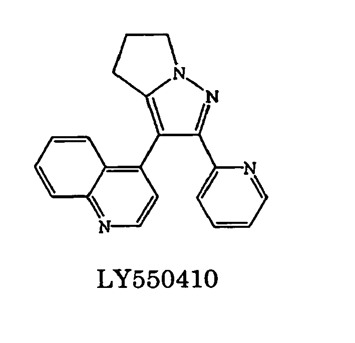

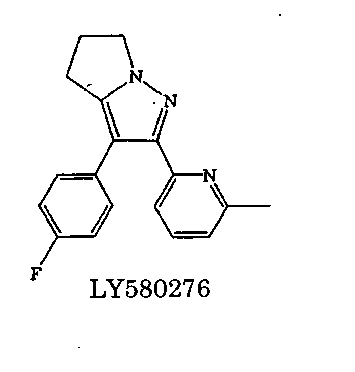

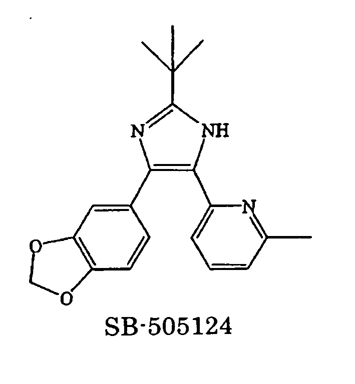

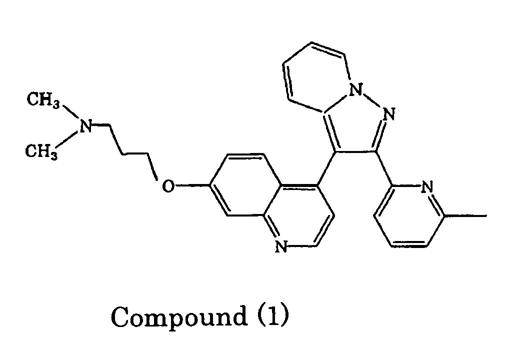

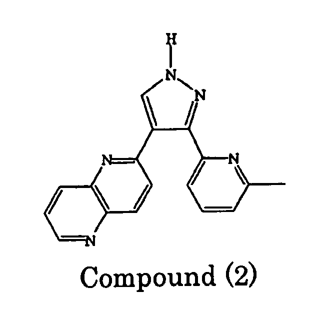

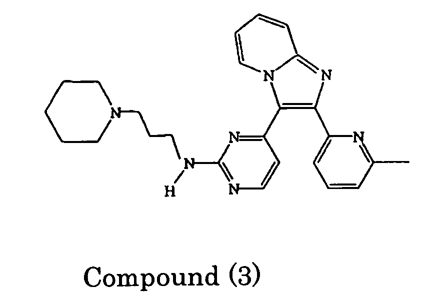

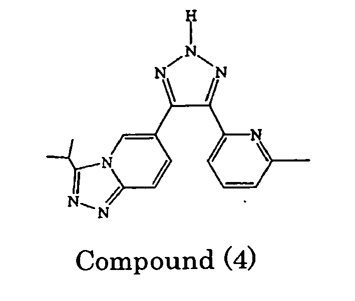

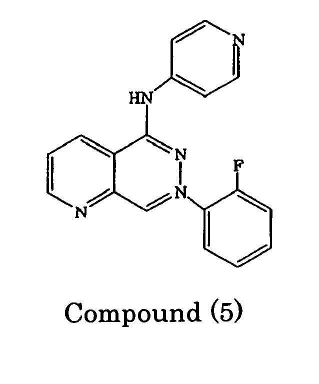

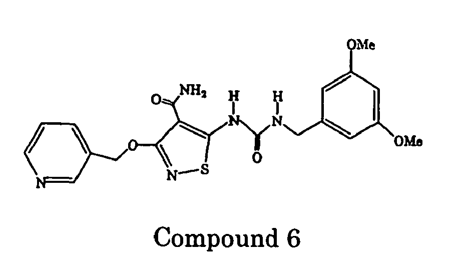

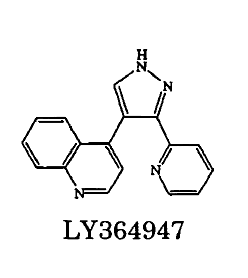

For information about these scaffolds and analogs thereof, Non-patent Reference 16 can be referred to regarding LY550410 and LY580276, which belong to dihydropyrrolopyrazole-based scaffolds, Byfield, S.D.et al., Mol.Pharmacol., 65, 744-752 (2004) can be referred to regarding SB-505124, which belongs to imidazole-based scaffolds, W02004/026871 can be referred to regarding Compound (1), which belongs to pyrazolopyridine-based scaffolds, Gellibert, F.et al., J.Med.Chem. 47,4494-4506 (2004) and Non-patent Reference 16, respectively, can be referred to regarding Compound (2) and LY364947, which belong to pyrazole-based scaffolds, WO2004/021989 can be referred to regarding Compound (3), which belongs to imidazopyridine-based scaffolds, WO2004/026307 can be referred to regarding Compound (4), which belongs to triazole-based scaffolds, WO2000/012497 can be referred to regarding Compound (5), which belongs to pyridopyrimidine-based scaffolds, WO2004/147574 can be referred to regarding Compound (6), which belongs to isothiazole-based scaffolds, and, in addition, Patent Reference 4 can be referred to regarding those that are pyrrolopyrazole-based. By quoting these references, the descriptive contents thereof in their entirety become the contents of the present specification. In addition, Non-patent Reference 2, 5 and 17 can also be referred to regarding related substances thereof.

In addition, modified referred to in "antitumor active substance modified so as to improve delivery capability to a tumor cell or a tumor tissue", which can be used in combination with the above TGF- β signaling inhibitor, does not refer to a substance comprising an original antitumor active substance or antitumor agent or anticancer agent in initial form that has been, for instance, merely turned into a simple salt, or turned into a simple prodrug, but means a substance in a form in which the delivery capability of the original antitumor active substance to a tumor cell or a tumor tissue has been improved using a carrier substance, a specific high molecular weight substance, and the like. Here, a form in which the delivery capability has been improved means a structure such that a drug is delivered efficiently overall to the targeted tumor cell or tumor tissue (for instance, a structure having increased resistance against degradation by various enzymes, or the like, in a living organism, increased stability in an aqueous solvent, or increased target selectivity using an antibody or the like, or altered solubility, and the like). Such modified antitumor active substances include, although not limiting, those in the form of a conjugate of a high molecular weight carrier suitable for stabilizing an antibody or a drug (in particular, drugs such as poly- or oligo-nucleotides, which are degraded by enzymes inside a living organism upon take-up by a specific cell) and an antitumor active substance (including complexes in which the carrier and the active substance have been bound with a covalent bond), the form in which an antitumor active substance has been encapsulated in a liposome or a macromolecular micelle, the form of a polymer vesicle and the form of a nanobubble. All are prepared according to modification techniques that are well known in the relevant technical field and thereto.

As such conjugates, although not limiting, complexes with a cationic charged polymer or a polymer or a copolymer holding a cationic charged polymer segment and an oligo- or polynucleotide (including single strand or double strand) or a derivative thereof and conjugates between an antibody or an inactive polymer such as poly(ethylene glycol) and an antitumor active substance covalently bonded via a hydrazone bond or a disulphide bond, which are cleavable *in vivo,* can be cited. Liposomes having an antitumor active substance in encapsulated form are formed with lipids or cationic lipids as a carrier, then, for macromolecular micelles in which the antitumor active substance is in encapsulated form, constructs formed with a block copolymer comprising a hydrophilic polymer chain segment and a hydrophobic polymer chain segment, and a block copolymer comprising a hydrophilic polymer chain segment and a charged polymer segment, as carriers, can be cited. As more specific [examples of] such copolymers, although not limiting, those in which the hydrophilic polymer chain segment is derived from poly(ethylene glycol), the hydrophobic polymer chain segment is derived from a polymer selected from the group consisting of poly(hydrophobic amino acid), poly(aspartic acid ester) and poly(glutamic acid ester), poly(lactide), poly(lactone) and copolymers thereof, the charged polymer segment is derived from a polymer selected from the group consisting of poly(glutamic acid), poly(aspartic acid), poly(lysine), poly(N,N-dialkylaminoalkyl(meta)acrylate) and poly(ethyleneimine) can be cited. The above poly(lactide) preferably includes poly(lactic acid) and poly(glycolic acid) obtained by ring-opening polymerization of lactides, poly(lactone) preferably include those obtained by ring-opening polymerization of β-lactose, γ-lactone, δ-lactone or ε-lactone. These copolymers may be modified to become temperature sensitive by integrating into the polymer chain unit, for instance, a unit derived from isobutylvinyl, or the like.

Meanwhile, the antitumor active substances usable in the present invention include any substances having antitumoral activity and modifiable as described above. As more specific [examples of] such antitumor active substances, although not limiting, oligo- or polynucleotides and derivatives thereof functioning either to inhibit the expression of an oncogene or to express a cancer suppressor gene (for instance, antisense or sense oligonucleotides, ncRNAs such as short interfering (siRNAs) and miRNAs, aptamers and decoy nucleic acids of a suitable gene (BCL2, clusterin, PKC α, methyltransferase, survivin, STAT3, HSP27, HRAS, KRAS2 and the like)), and antibodies (binding to tumor cell or tumor tissue specifically), as well as antimetabolites (for instance, fluorouracil, tegafur, carmofur, doxifluridine, cytarabine, cladribine, gemcitabine, methotrexate and the like), alkylating drugs (for instance, cyclophosphamide, nimustine and the like), platinum formulations (carboplatin, cisplatin, nedaplatin, oxaliplatin, diaminocyclohexane platinum (II) and the like), antibiotics (mitomycin C, aclarubicin, epirubicin, doxorubicin (or adriamycin), pirarubicin, mitoxantrone, bleomycin and the like), and plant extracts (for instance, vinorelbine, vincristine, vindesine, vinblastine, docetaxel, paclitaxel (or taxol), etoposide, irinotecan and the like) can be cited. Note that, as can be understood from the above, antibiotics and plant extracts referred to in the present invention are limited to those having antitumoral activity, in addition, include compounds corresponding to derivatives of compounds obtained from nature. In addition, it is understood that radioactive nuclides used in radiation therapy are included in the above-mentioned antitumor active substances.

Among the macromolecular micelles or conjugates encapsulating such antitumor active substances, as ones of interest containing those [substances] supplied to clinical trials, macromolecular micelle from a poly(ethylene glycol) block-poly(aspartic acid) copolymer encapsulating adriamycin (for instance, refer to Patent Reference 1), macromolecular micelle from a poly(ethylene glycol) block-poly(aspartic acid ester) copolymer encapsulating paclitaxel (for instance, Hamaguchi,T., et al., Br.J.Cancer, 92: 1240-1246, 2005), macromolecular micelle from a poly(ethylene glycol) block-poly(glutamic acid) copolymer encapsulating cisplatin or diaminocyclohexane platinum (II) (for instance, refer to WO02/26241 A1 and W02005/056641 A1), macromolecular micelle from a poly(ethylene glycol) block-poly(aspartic acid ester) copolymer encapsulating camptotecin or a derivative thereof, for instance topotecan (for instance, refer to WO03/099260 A1 and W02005/023230), and the like, can be cited. As conjugates of an antibody with a drug that are usable in the present invention, although not limiting, a conjugate of the CD33 antibody, which is an antibody that may bind specifically to tumor cell, with calicheamicin (gemtuzumab ozogamycin: refer to US 5,773,001 B) may be cited, and as conjugates of an inactive high molecular weight compound (polyethyleneglycol (sometimes abbreviated as PEG), albumin and the like) with a drug, PEG-interferon (for instance, Wang,Y.S. et al., Adv. Drug Delv. Rev. 54,547-570, 2002), PEG-anti-TNF Fab conjugate (Chapman, A.P. et al., Nature Biotechnol. 17,780-783, 1999) and the like may be cited, and in addition, conjugates described in Duncan, R. Nature Reviws Drug Discovery 2,347-360, 2003 can be cited (by citing the above references, the contents thereof in their entirety become the contents of the present specification). In addition, regarding examples in which an oligonucleotide has been encapsulated by a macromolecular micelle, Kakizawa, Y.et al., Biomacromolecules 2001, 2,491-497 can be referred to.

According to the present application, using the action of increasing the leakiness of neovasculature in tumor tissue exerted by the above TGF-β signaling inhibitor, a system for delivering selectively to a tumor tissue various imaging agents can also be provided. The imaging agents (or various contrast reporter constituents: for instance, Tc, Gd, Mn and the like) used are preferably those that have been modified using high molecular weight substance (for instance, polyethyleneglycol, dendrimer and the like, which have been modified so as to confer bindability to an imaging agent) described regarding the above antitumor agents. Such imaging agents may be any ones as long as they suit the object of the present invention and, although not limiting, those mentioned in Marccos, E.et al., Bioconjugate Chem.1998,9,184 - 191; Torchilin V.P.Advanced Drug Delivery Reviews 54 (2002) 235-252; Kobayashi H. et al., Advanced Drug Delivery Reviews 57 (2005) 2271-2286 and in other references cited therein, can be cited (by citing these references, the descriptive contents thereof are incorporated herein).

The combination for tumor treatment according to the present invention combining the above TGF- β signaling inhibitor and the above antitumor agent or antitumor active substance, and in particular, modified antitumor active substance, allows the inhibitor and the antitumor active substance to exist combined within a single formulation, or to exist as separate formulations. In addition, when administering the TGF- β signaling inhibitor and the antitumor active substance to an individual (a mammal, and preferably a human), administration can be both parties simultaneously, or after administration of one, administration of the other agent with the observance of a suitable interval. In the latter case, in general, TGF-β signaling inhibitor is administered first, preferably. The ratio of these combinations cannot be specified since the optimal value differs depending on the type of TGF- β signaling inhibitor and modified antitumor active substance used; however, referring to the examples described later, in general, a small scale animal experiment can be carried out, and taking the result obtained therefrom as a reference, [the ratio] can be determined by a specialized physician. In addition, when they exist as separate formulations, they can be administered from an administration route that is identical or different from one another. As these administration routes, they can be oral administration, arterial injection, venous injection, intratumoral injection, subcutaneous injection, and the like. The dose of the antitumor active substance can be determined with the dose of the original drug as a reference.

The above administration of TGF- β signaling inhibitor is performed, in particular, into an individual, a mammal, and preferably a human, requiring an increase in leakiness without causing neovasculature to regress, using a therapeutic effective dose. Therapeutic effective dose can be a low dose that does not reach a cancer cell *per se,* as described above and explained in the examples below. Such dose can also be determined by a specialized physician, by referring to the examples described later, if necessary, further carrying out an animal experiment, and based on the result thereof. In addition, the therapeutic effective dose of an antitumor active substance, in the cases of drugs that are already in clinical use, can be determined by referring to the therapeutic effective doses thereof; however, in general, a dose that is the already-known dose or less can be chosen. Meanwhile, in the cases of drugs that are not in clinical use, a specialized physician can determine [the dose] after carrying out a suitable animal experiment.

As tumors mainly intended for treatment according to the present invention by a TGF- β signaling inhibitor or combination, tumor group considered to be resistant to chemotherapy due to the stromal components being abundant or of a poorly neovascularized tissue type, can be cited. However, when used in other tumors than these, a desirable efficacy is also obtained. As the above tumor group, intractable digestive organ tumors including pancreatic adenocarcinoma, scirrhous gastric cancer, cholangiocellular cancer, metastasizing hepatic tumor and the like, intractable soft tissue tumors including malignant fibrous histiocytoma (MFL) and fibrosarcoma, a portion of central nervous system tumors including medulloblastoma, a portion of breast cancers (in particular, with strong fibrosis) and the like, and breast cancers with increased fibrosis, can be cited.

According to the present invention, with respect to vasculature, using a TGF-β inhibitor that acts selectively only on an unstable neovasculature, the access of an anticancer agent to a tumor or cancer tissue where angiogenesis has been initiated can be improved specifically without altering the accumulation of the anticancer agent in a normal site where angiogenesis has not occurred. Therefore, when applied in combination with a TGF-β inhibitor, and, an antitumor active substance, the influence and adverse effects not only by the TGF- β inhibitor, but also by the antitumor active substance can be held at a minimum.

In the present invention, the above combination can be in the form of a kit for treating tumor, containing separately a medicinal formulation containing a TGF- β signaling inhibitor and a medicinal formulation containing a modified antitumor active substance.

### Brief Description of the Drawings

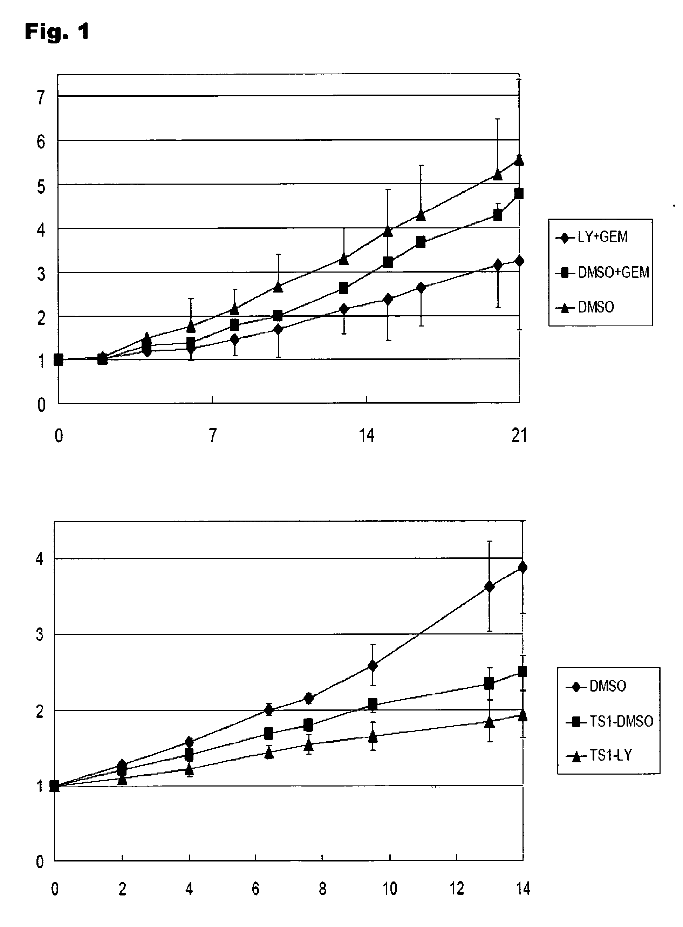

Fig. 1 shows graphs indicating the changes in tumor volume over time, representing the effects of the combined application of a general anticancer agent and a TGF- β signaling inhibitor in pancreatic adenocarcinoma cells, according to Example 1 described later. The vertical axis represents the relative tumor volume and the horizontal axis represents time (day);
Fig. 2-1 are photographs in lieu of figures showing immunofluorescence histological staining representing the effects of a TGF- β signaling inhibitor in a Matrigel plug assay, according to Example 2 described later. (a), (b-1) and (b-2), in this order, are the results for the control group, the LY-administered group and the TbRIIFc-administered group, respectively, with green showing PECAM-1 (vascular endothelial cell marker), red showing SMA (vascular pericyte marker), and blue showing cell nucleus staining, in all. (c) and (d) show the distribution in the Matrigel plug of dextran that has been injected intravenously into the tail, with green and red showing the same as above and blue showing dextran;
Fig. 2-2 shows graphs quantitatively representing the effects of the TGF- β signaling inhibitor in the Matrigel plug assay shown in Fig. 2-1, according to Example 2 described later. (a) shows the rates of coverage of vascular endothelial cells by vascular pericytes, (b) shows the total areas of vascular endothelium marker-positive portion and (c) shows the dextran distribution areas per one microscope visual field. The error bars show standard errors;

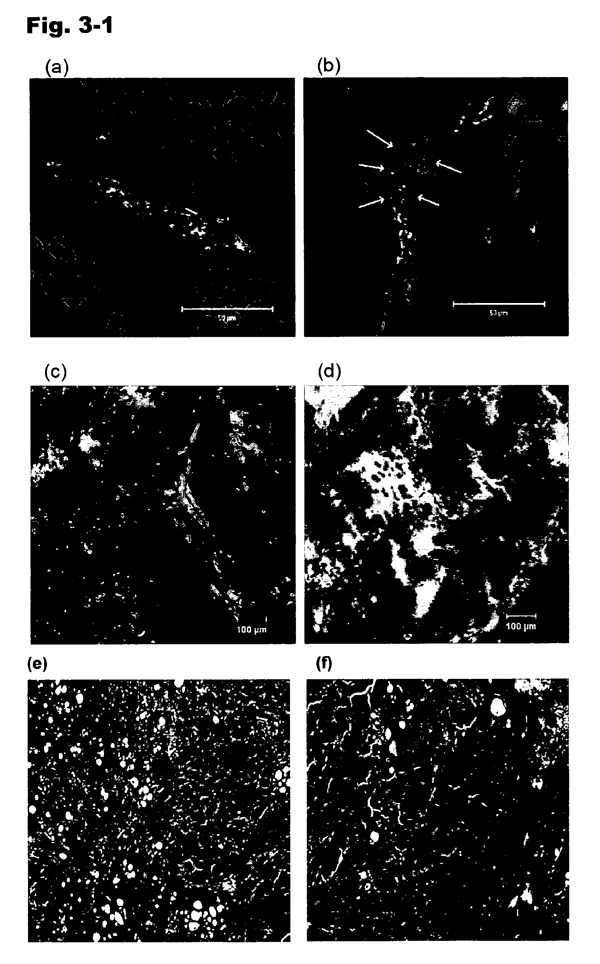

Fig. 3-1 are photographs in lieu of figures showing the effects of a TGF- β signaling inhibitor in tissues of a BxPC3 cell xenograft model, according to 3-1 of Example 3 described later. The left column (a, c and e) is for control conditions and the right column (b, d and f) is for LY-administered conditions. (a and b) show the morphology of blood vessels in cancer tissue, with green showing VE-cadherin (vascular endothelium marker), red showing NG2, and purple color showing staining of multiplying nuclei (Ki-67). (c and d) show the accumulation of systemically administered dextran into cancer tissue. Green is dextran and blue is cell nucleus staining. (e and f) are specimens in which Azan staining was performed, which stains extracellular fibrous components in blue;

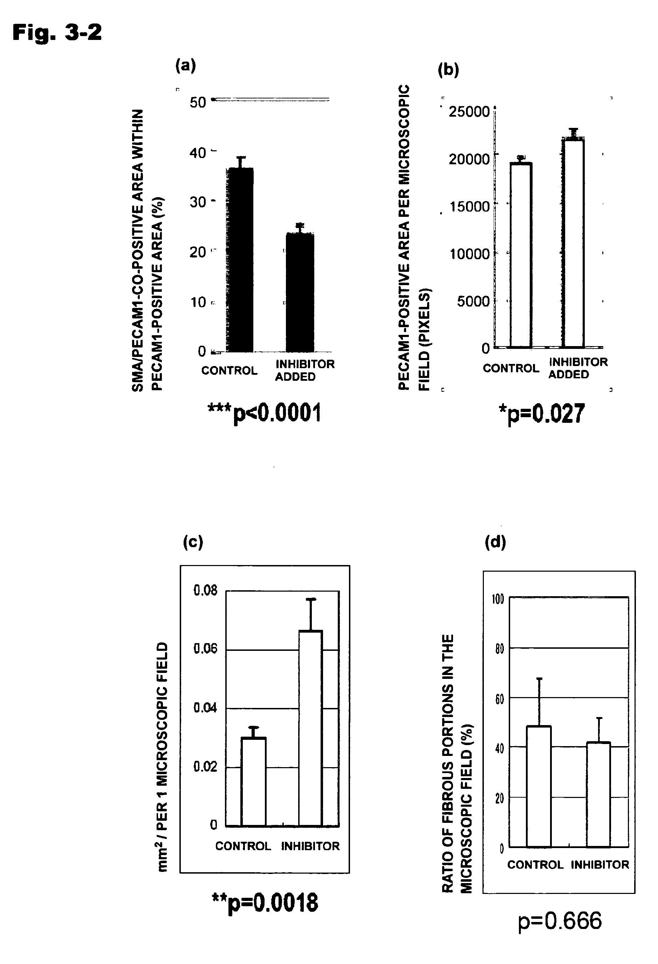

Fig. 3-2 shows graphs quantitatively representing the effects of a TGF- β signaling inhibitor in tissues of a BxPC3 cell xenograft model, according to 3-1 of Example 3 described later. (a) shows the rates of coverage of vascular endothelial cells by vascular pericytes, (b) shows the total areas of vascular endothelium marker-positive portion, and (c) shows the dextran distribution areas per one microscope visual field. The error bars show standard errors. (d) shows the proportion of fibrous components per one microscope visual field;

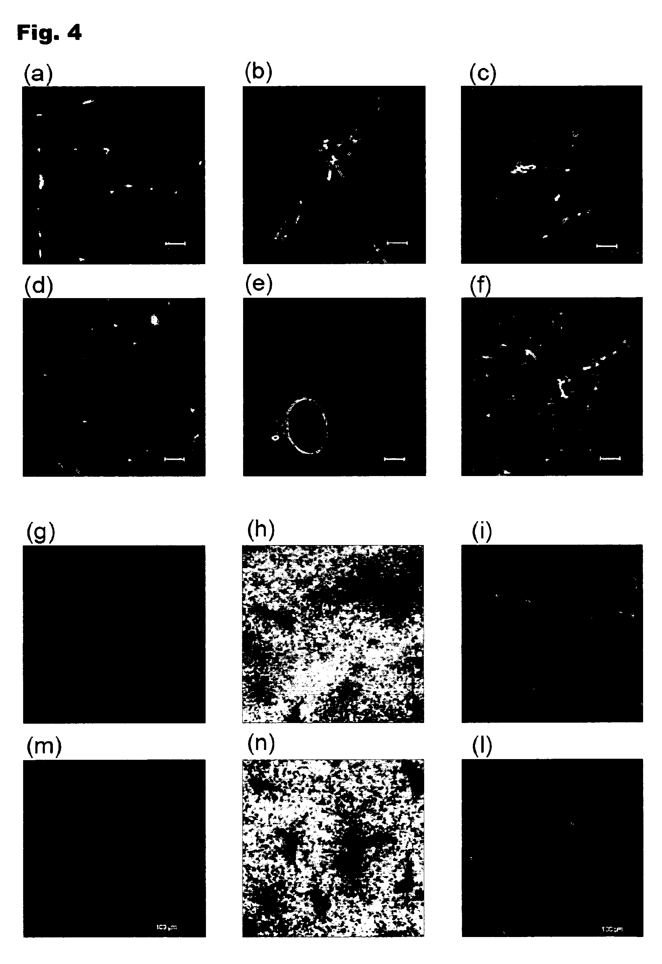

Fig. 4 are photographs in lieu of figures showing the effects of a TGF-β signaling inhibitor in blood vessels in general organs, according to 3-2 of Example 3 described later. The left column (a, d, g and m) is for brain, the middle column (b, e, h and n) is for liver, and the right column (c, f, i and 1) is for kidney. The upper level (a-f) is for blood vessel staining, with green showing PECAM-1, red showing SMA, and blue showing cell nucleus staining. Among these, (a-c) are for the control group and (d-f) are for the LY-administered group. In addition, the lower level (g-1) is for the distribution of dextran after administration, with green showing dextran and blue showing cell nuclei;

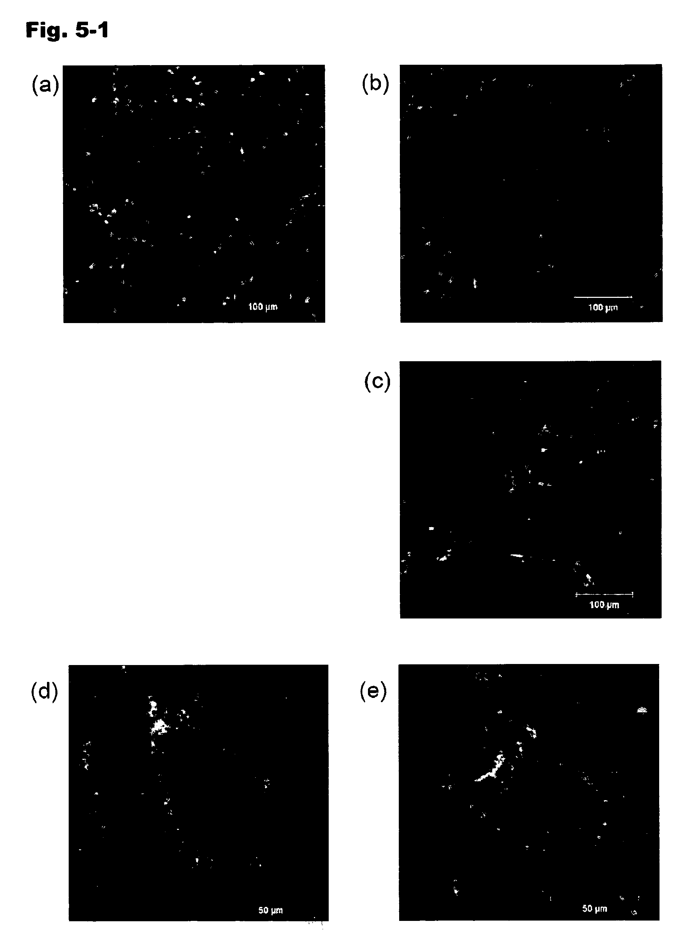

Fig. 5-1 are photographs in lieu of figures showing the determination of drug efficacy range of a TGF- β signaling inhibitor, according to 3-3 of Example 3 described later. Green is phosphorylated Smad2 (if positive, strongly suggests signal transduction of TGF- β occurred), red is PECAM-1 and blue is cell nucleus staining. (a and c) are visual fields at weak magnification, (a) being the control condition, (b) being LY 1mg/kg and (c) being LY 25mg/kg condition. (d and e) are visual fields at strong magnification focused on blood vessels;

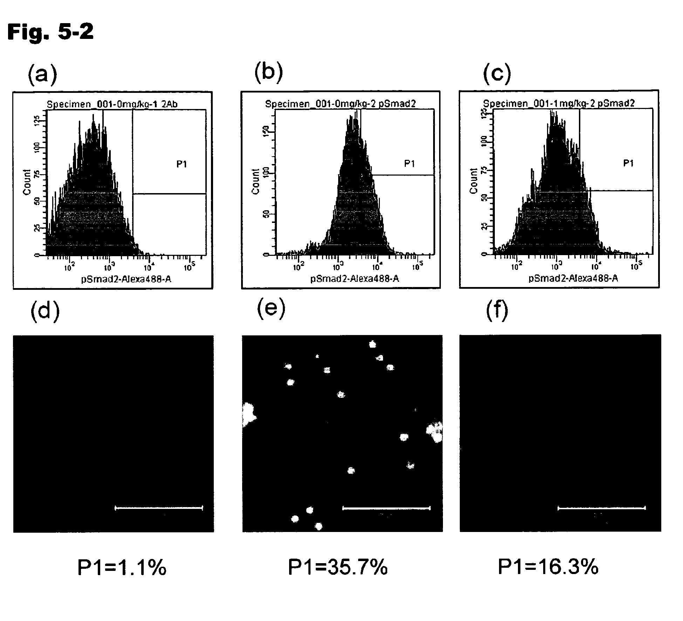

Fig. 5-2 shows the effects of a TGF- β signaling inhibitor on nucleated blood cells, according to 3-4 of Example 3 described later. The upper level (a-c) [show] frequency distributions by cell sorter of phosphorylated Smad2-positive cells among peripheral blood nucleated cell, and the lower level (d-f) are photographs in lieu of figures showing smear images of the specimens at that time. The leftmost (a) is a control (for staining) with the secondary antibody only, which is where the gate was set so as to obtain P1=1%. Using the same gate setting, the proportions of (b) non-LY-administered group and (c) LY-administered group in the P1 gate were determined;

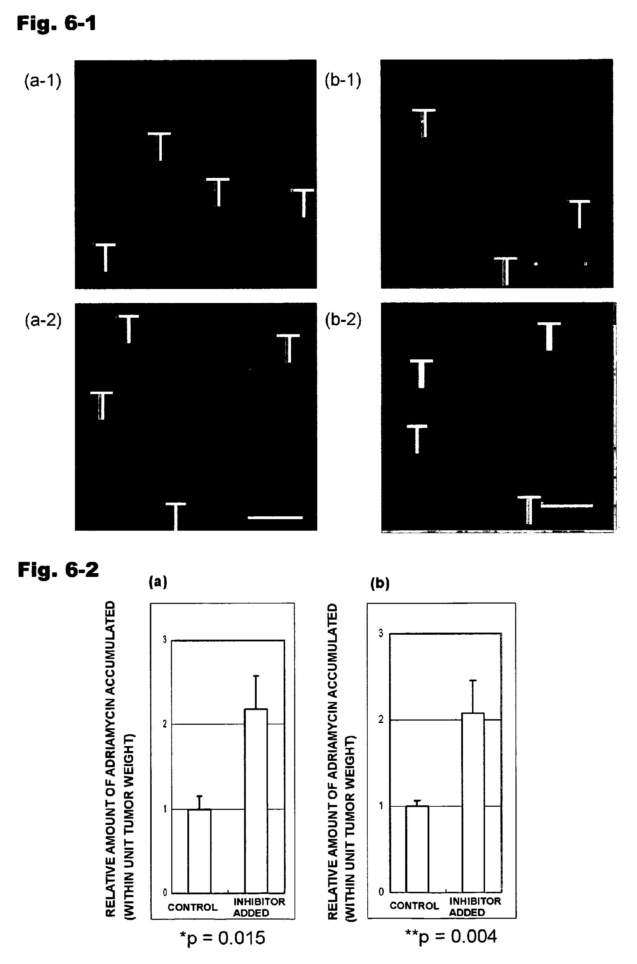

Fig. 6-1 are photographs in lieu of figures showing the distribution of a macromolecular anticancer agent in cancer tissue when a TGF- β signaling inhibitor was administered in combination in BxPC3 subcutaneous tumor model, according to 3-5 of Example 3 described later. T's show the tumor cell portions and the other locations are stroma in tumor tissue. The left column (a-1 and 2) is distribution of Doxil, and the right column (b-1 and 2) is distribution of Micelle-ADR. The upper level (a-1 and b-1) is for the control group and the lower level (a-2 and b-2) is for the LY combined application group;
Fig. 6-2 shows the accumulation of a macromolecular anticancer agent in cancer tissue measured by the HPLC method, when a TGF- β signaling inhibitor was administered in combination in a BxPC3 subcutaneous tumor model, according to 3-5 of Example 3 described later. (a) is the amount of Doxil accumulated and (b) is the amount of Micelle-ADR accumulated;

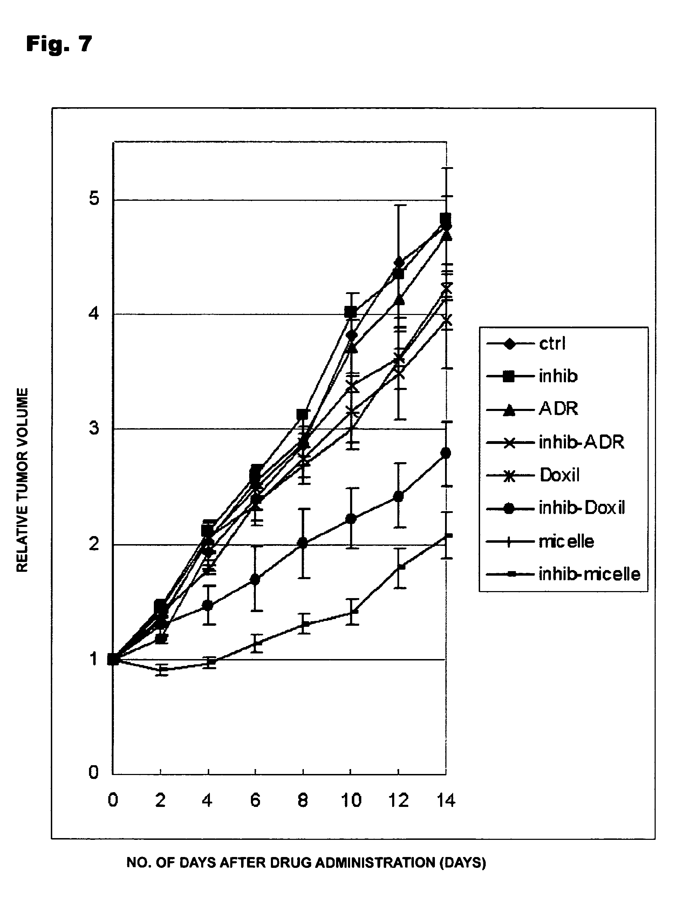

Fig. 7 is a graph representing chronological tumor growth curve when ADR, Doxil and Micelle-ADR were used on BxPC3 by lone administration, according to 3-6 of Example 3 described later. The vertical axis represents the relative tumor volume and the horizontal axis represents time (days);

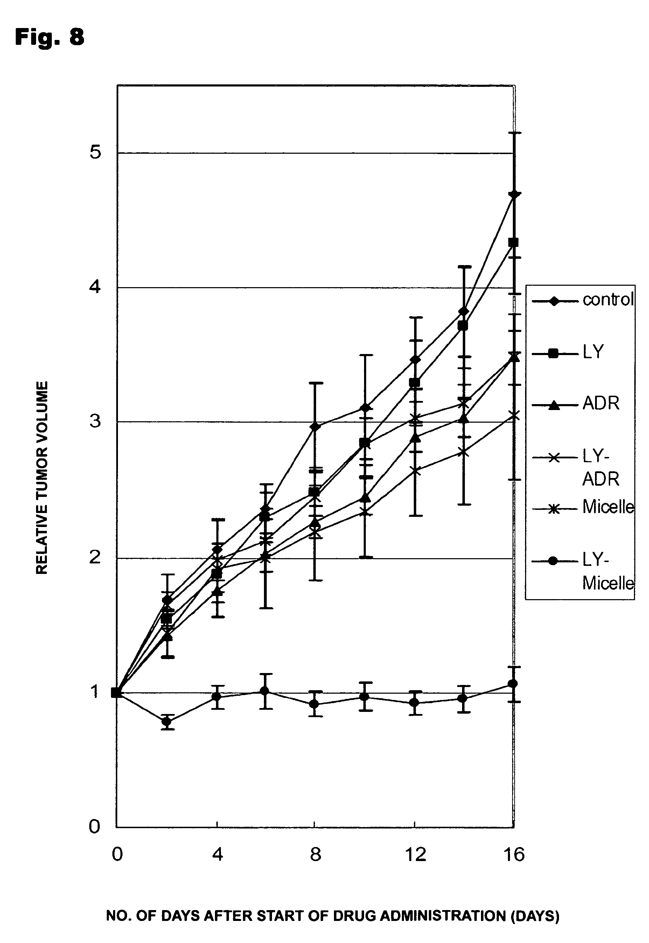

Fig. 8 is a graph representing chronological tumor growth curve when ADR and Micelle-ADR were used on BxPC3 by administering three times spaced by four days, according to 3-7 of Example 3 described later. The vertical axis represents the relative tumor volume and the horizontal axis represents time (days);

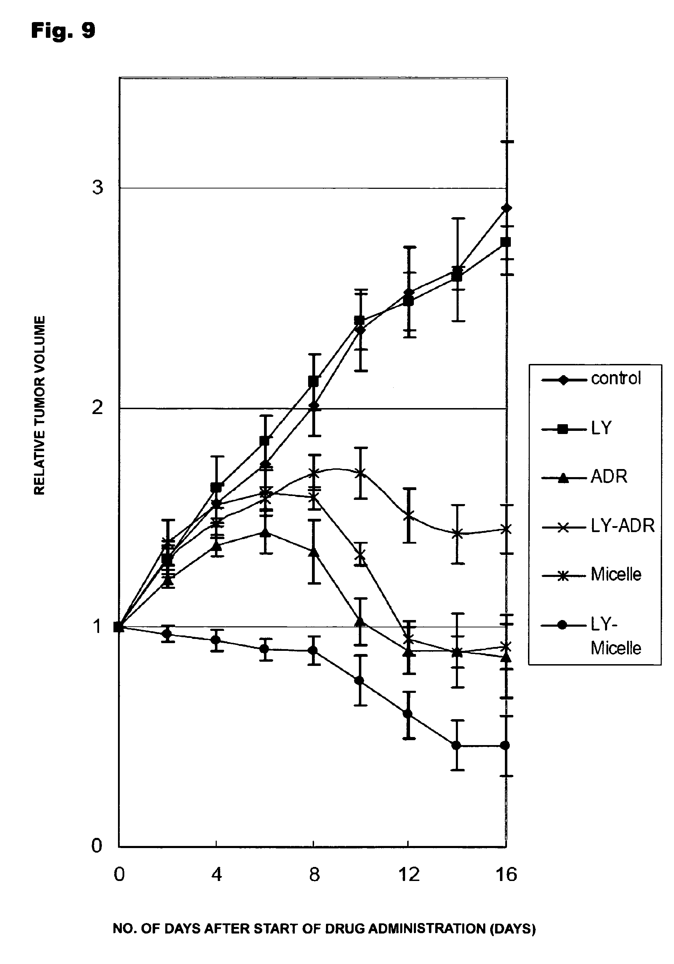

Fig. 9 is a graph representing chronological tumor growth curve when MIA PaCa-2-drug agent was used, according to 3-8 of Example 3 described later. The vertical axis represents the relative tumor volume and the horizontal axis represents time (days);

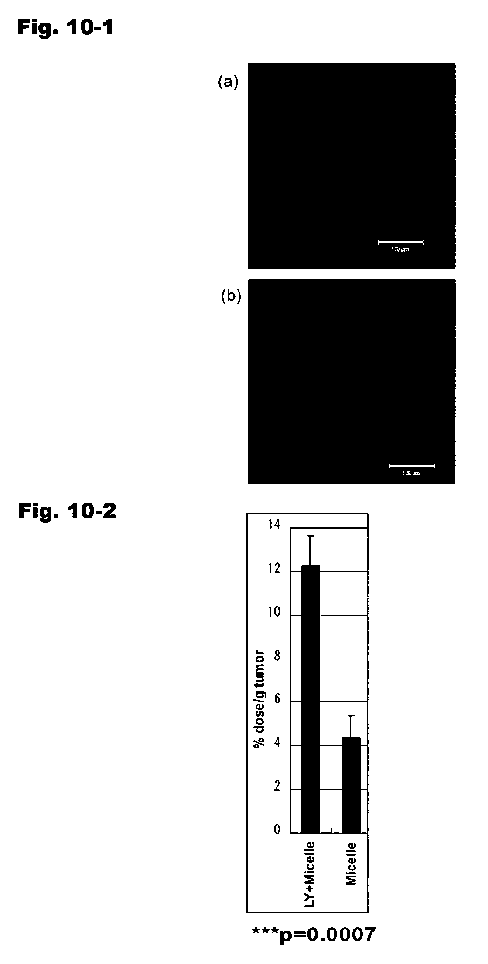

Fig. 10-1 are photographs in lieu of figures showing distribution of Micelle-ADR in cancer tissue when a TGF-β signaling inhibitor was administered in combination in a MIA PaCa-2 subcutaneous tumor model, according to 3-9 of Example 3 described later. The upper level (a) is for the LY-administered group and the lower level (b) is for the control group;
Fig. 10-2 shows the accumulation of a macromolecular anticancer agent in cancer tissue measured by the HPLC method, when a TGF- β signaling inhibitor was administered in combination in a MIA PaCa-2 subcutaneous tumor model, according to 3-9 of Example 3 described later;

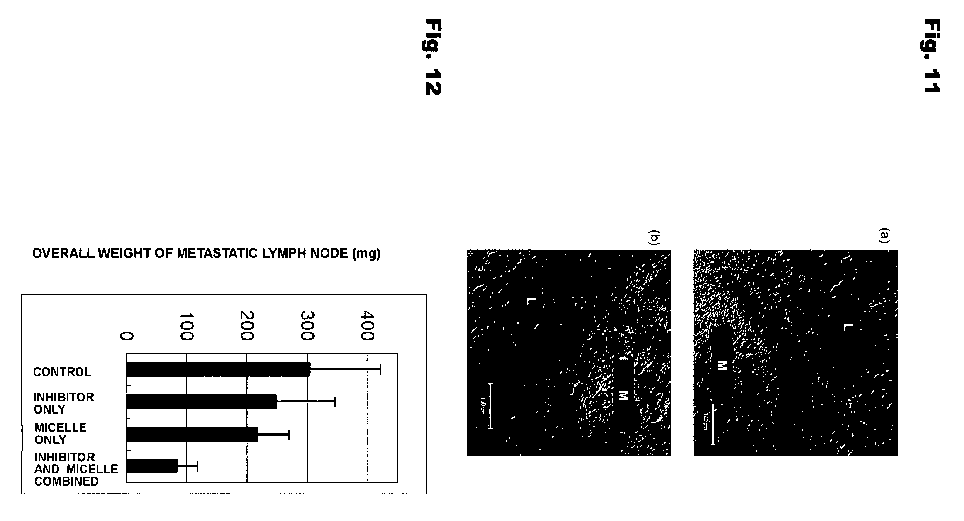

Fig. 11 are photographs in lieu of figures showing the distribution of Micelle-ADR in cancer tissue when a TGF-β signaling inhibitor was administered in combination in a model of liver metastatic foci from pancreatic cancer obtained in a MIA PaCa-2 orthotopic graft model, according to 3-10 of Example 3 described later. (a) is for the control group and (b) is for the LY combined application group. L indicates normal liver tissue, M and indicates metastatic foci. Noteworthy is the remarkable accumulation effect by the combined application of LY in M, in contrast to the extent of accumulation almost not changing in L;
Fig. 12 represents the total weight of involved lymph node obtained in an OCUM-2MLN orthotopic graft model, according to 3-11 of Example 3 described later;

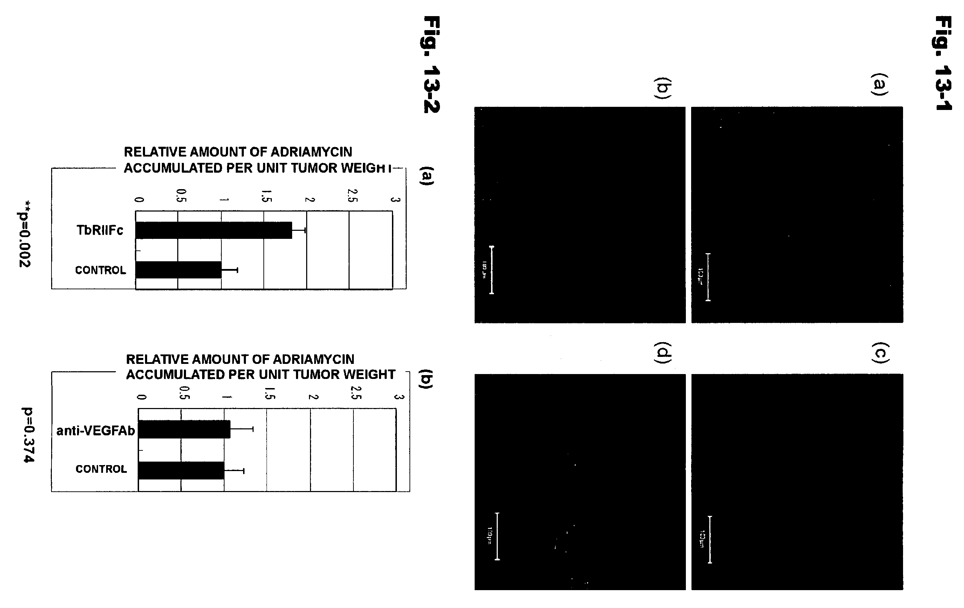

Fig. 13-1 are photographs in lieu of figures showing the distribution of a macromolecular anticancer agent in cancer tissue when a TGF-β signaling inhibitor other than LY (TbRIIFc, (a and b)) or a VEGF inhibitor (anti-VEGF antibody, (c and d)) was administered in combination in BxPC3 subcutaneous tumor model, according to 3-12 of Example 3 described later;
Fig. 13-2 shows the accumulation of a macromolecular anticancer agent in cancer tissue when a TGF- β inhibitor other than LY (TbRIIFc, (a)) or a VEGF inhibitor (anti-VEGF antibody, (b)) was administered in combination in a BxPC3 subcutaneous tumor model, according to 3-13 of Example 3 described later;

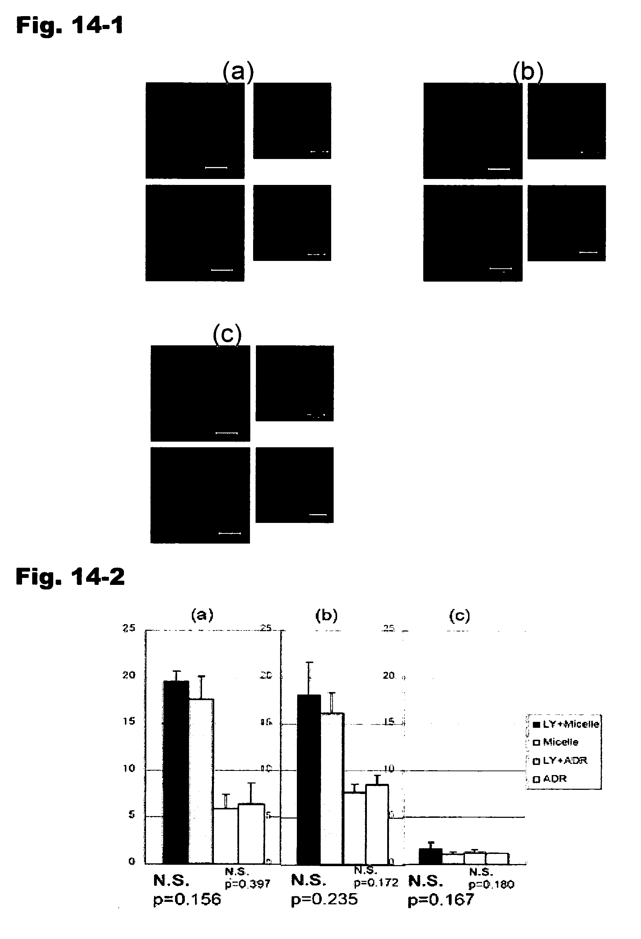

Fig. 14-1 are photographs in lieu of figures showing distribution in general organs in a combined administration of a TGF- β signaling inhibitor and Micelle-ADR or free ADR, according to 3-14 of Example 3 described later. With respect to liver (a), kidney (b) and spleen (c), the upper level represents the LY-administered group, the lower level represents the control group, the left column represents Micelle-ADR and the right column represents free ADR; and
Fig. 14-2 shows the amount of accumulation in general organs in a combined administration of a TGF-β signaling inhibitor and Micelle-ADR or free ADR, according to 3-14 of Example 3 described later. With respect to liver (a), kidney (b) and spleen (c), inhibitor and micelle combined application, micelle alone, inhibitor and free ADR combined application, and free ADR alone are arranged respectively from the left. N.S. in the numbers indicated below means not significant statistically.

### Best Mode for Carrying Out the Invention

### <Examples>

Hereinafter, the present invention will be described more concretely giving concrete examples; however the present invention is not intended to be limited to these embodiments.

### Example 1: Combined effect of a general anticancer agent (unmodified) and a TGF-β signaling inhibitor in pancreatic adenocarcinoma model

### Drug: Gemcitabine

### Method:

Four weeks-old male nude mice were subcutaneously injected in the abdominal wall with 5×10⁶ counts of Smad4-deficient pancreatic adenocarcinoma cells (BxPC3: ATCC No.CRL-1687), and when approximately two weeks have elapsed from the transplantation, those in which the cancer cell mass entered a proliferative phase were used in the experiment as xenograft models of pancreatic adenocarcinoma. Combining Gemcitabine (hereinafter, abbreviated as Gem), administrated twice a week intraperitoneally at 125 mg/kg, and LY364947 (hereinafter, referred to as LY), administrated three times a week intraperitoneally at 1 mg/kg, three groups were established as follows: a control group, a Gem alone administration group and a LY+Gem combined application group, with n=2 each. For these animals, the values of the tumor volume were observed for 21 days. The tumor volumes were approximated by long side of tumor mass × (short side)² and compared, taking 1 as the value at the administration start time for each tumor mass. The result is shown in the graph at the upper level of Fig. 1.

### Result:

On the last day, the combined application group exhibited a shrinkage of the tumor compared to the singly administrated group, with 5.56±1.59 for the control group, 4.76±0.46 for the Gem alone administration group, and 3.25±1.15 for the LY+Gem combined application group.

### Drug: TS-1

### Method:

Four weeks-old male nude mice were subcutaneously injected in the abdominal wall with 5×10⁶ counts of Smad4-deficient pancreatic adenocarcinoma cells (BxPC3), and when approximately two weeks have elapsed from the transplantation, those in which the cancer cell mass entered a proliferative phase were used in the experiment as xenograft models of pancreatic adenocarcinoma. Combining oral administration of 5 mg/kg of TS-1 (peroral 5FU prodrug, Taiho Pharmaceutical) daily, and LY administrated three times a week intraperitoneally at 1 mg/kg, three groups were established as follows: a control group, a TS-1 alone administration group and a LY+TS-1 combined application group, with n=3 each. For these animals, the approximated values of the tumor volume were observed for 14 days. For the tumor volumes, those that have been approximated by long side of tumor mass × (short side)² were compared, taking 1 as the value at the administration start time for each tumor mass.

### Result:

On the last day, the LY+TS-1 combined application group displayed a shrinkage of the tumor compared to the TS-1 alone administration group, with 3.88±0.61 for the control group, 2.49±0.23 for the TS-1 alone administration group and 1.93±0.31 for the LY+TS-1 combined application group. The evolution is shown in the graph of Fig. 1. The result is shown in the graph at the lower level of Fig. 1.

### Example 2: Effects of TGF- β signaling inhibitor on neovasculature by Matrigel plug assay

### Method:

VEGF-A, FGF-2, and heparin were mixed into Matrigel (BD), furthermore, 500nM of the TGF- β inhibitor LY364947 (hereinafter, referred to as LY) or 50 µg/ml of T βRII:Fc or a control was further mixed into the same gel, these were subcutaneously injected into the abdominal wall of male ICR mice, the animals were sacrificed on the 7th day to extract the Matrigel plug, and a search was carried out using immunofluorescence staining. Concretely, regarding the neovasculature morphology, vascular endothelial cells were recognized with an antibody against PECAM-1 (platelet endothelial cell adhesion molecule-1) and vascular pericytes were recognized with an antibody against SMA (α smooth muscle actin), in the LY-administered group or control group, the proportion of the areas covered by pericytes within the total endothelial cell area, and, the areas positive for the vascular endothelium marker were quantified (hereabove, n=20). In addition, 6 hours prior to sacrifice, macromolecular dextran with a molecular weight of 2,000,000 (Invitrogen Molecular Probes) was administered via the tail vein, and the difference in distribution in tumor tissue due to the presence or absence of inhibitor was investigated by quantification of the distribution area (n=21).

### Result:

The result is shown in Fig. 2-1 and Fig. 2-2. The phenotypes of the LY-administered group and the TbRIIFc-administered group were fundamentally equivalent. The endothelial cell coverage of the pericytes decreased significantly for the LY-administered group compared to the non-administered group (p=0.006). In addition, the total area of the vascular endothelium, increased significantly for the LY-administered group (p=0.0009). The dextran distribution area increased approximately two-fold due to LY administration, and this difference was significant (p<0.0001).

### Example 3: Combined application of macromolecular compound and TGF-β signaling inhibitor

### 3-1. Smad4-deficient TGF-β signaling-non-responsive pancreatic adenocarcinoma cell (BxPC3)-high molecular weight dextran-LY364947, neovasculature morphological change and dextran distribution

### Method:

Four weeks-old male nude mice were subcutaneously injected in the abdominal wall with 5×10⁶ counts of Smad4-deficient pancreatic adenocarcinoma cells (BxPC3), and when approximately two weeks have elapsed from the transplantation, those in which the cancer cell mass entered a proliferative phase were used in the experiment as xenograft models of pancreatic adenocarcinoma. Twenty-four hours before sacrificing the animals, 1mg/kg of LY364947 (hereinafter, referred to as LY) as TGF- β inhibitor or a control was administered once intraperitoneally. The animals were sacrificed to extract the tumor mass, and a histological search was performed. Concretely, regarding the neovasculature morphology, vascular endothelial cells were recognized with an antibody against PECAM-1 (platelet endothelial cell adhesion molecule-1), vascular pericytes were recognized with an antibody against NG2 (neuroglycan 2), the proportion of the areas covered by pericytes within the total endothelial cell area, and ,the areas positive for the vascular endothelium marker were quantified (hereabove, n=4 animals × 10 microscopic visual fields =40), in addition, regarding dextran distribution, the distribution area thereof was quantified. In addition, after an identical model was prepared, 6 hours prior to sacrifice, macromolecular dextran with a molecular weight of 2,000,000 (Invitrogen Molecular Probes) was administered via the tail vein, and the difference in distribution in tumor tissue due to the presence or absence of inhibitor was investigated by quantification of the distribution area (n=3 animals × 4 microscopic visual field =12).

### Result:

The extent of coverage of endothelial cells by the pericyte was 23.3±1.9% in the presence of TGF- β inhibitor, in contrast to 36.0±2.2%, which was for the control (p=0.00002 by the t-test). Next, endothelial cell marker-positive area was 1.13±0.06 in the presence of TGF- β inhibitor, in contrast to 1.00±0.03 for the control (p=0.02 by the t-test). In addition, macromolecular dextran distribution area was 2.24±0.36 in the presence of TGF-β inhibitor in contrast to 1.00±0.13 for the control (p=0.002 by the t-test). (Refer to Fig. 3-1 and Fig. 3-2)

Note that in condition where this LY dose was 1mg/kg, in azan staining, which stains collagen fibers separately, no significant difference in the fibrosis of tumor tissue was observed between LY-administered and non-administered groups (P=0.336 by quantification of the areas on the specimens).

### 3-2. General organs-high molecular weight dextran-LY364947, vasculature morphological change and dextran distribution

### Method:

Brain, liver and kidney were extracted from animals used in 3-1 to carry out histological search. Regarding vasculature, vascular endothelial cells were labeled with an antibody against PECAM-1, vascular pericytes were labeled with an antibody against SMA (Smooth muscle actin), and, in addition, dextran distribution was observed.

### Result:

No clear change in morphology accompanying TGF- β signaling inhibition was observed in the existing vasculature of these general organs. In addition, no clear difference was observed in the dextran distribution. (Refer to Fig. 4)

### 3-3. Observation of TGF- β signaling inhibition effect in cancer tissue one hour after administration of LY364947 by staining of phosphorylated Smad2

### Method:

Four weeks-old male nude mice were subcutaneously injected in the abdominal wall with counts 5×10⁶ of Smad4-deficient pancreatic adenocarcinoma cells (BxPC3), and when approximately two weeks have elapsed from the transplantation, those in which the cancer cell mass entered a proliferative phase were used in the experiment as xenograft models of pancreatic adenocarcinoma. As TGF- β inhibitor, 1mg/kg, 25mg/kg or a control of LY was administered once intraperitoneally. The animals were sacrificed one hour after administration to extract the tumor, and immuno-histological staining of the tumor was carried out using an antibody against phosphorylated Smad2 as well as an antibody against PECAM1.

### Result:

When 1mg/kg of LY was administered, no remarkable difference was observed compared to the control, in a weak magnification microscopic visual field (refer to Fig. 5-1 (a) and (b)). However, when intratumoral vascular walls were observed in a magnified visual field, cancellation of Smad2 phosphorylation was observed also at this dose. Meanwhile, when 25mg/kg of LY was administered, cancellation of Smad2 phosphorylation was observed in almost the entirety of the cancer cells. (Refer to Fig. 5-1)

### 3-4. Observation of TGF- β signaling inhibition effect in nucleated blood cells one hour after administration of LY364947 by staining of phosphorylated Smad2

### Method:

Cancer-transplanted nude mice were administered once intraperitoneally with 1mg/kg of LY as TGF- β inhibitor or a control. One hour after administration, whole blood collection was carried out, hemolysis treatment, formalin fixation, cell membrane perforation by methanol were carried out, then, staining was carried out with an antibody against phosphorylated Smad2, thereafter, the ratio of phosphorylated Smad2-positive cells was measured by FACS. In so doing, with those [samples] where only the secondary antibody was reacted serving as controls for the immuno-staining method, the gate was set so that rate of positive cell was approximately 1% in this group (P1 in Fig. 5-2), and the ratio of phosphorylated Smad2-positive cells was measured for the TGF- β inhibitor-administered group and the non-treated group.

### Result:

Inhibition of Smad2 phosphorylation was observed, with 35.7% of cells being positive for phosphorylated Smad2 in the control group, which contrasts with 16.3% positive in the LY-administered group. From 1-3 and 1-4, observation of TGF- β signal inhibition was suggested in vascular wall and blood cells, even with a dose of 1mg/kg of LY (refer to Fig. 5-2).

### 3-5. Smad4-deficient pancreatic adenocarcinoma cell (BxPC3) - macromolecular formulation (Doxil and micelle adriamycin) - tissue distribution

### Method:

Four weeks-old male nude mice were subcutaneously injected in the abdominal wall with 5×10⁶ counts of Smad4-deficient pancreatic adenocarcinoma cells (BxPC3), and when approximately two weeks have elapsed from the transplantation, those in which the cancer cell mass entered a proliferative phase were used in the experiment as xenograft models of pancreatic adenocarcinoma. As a macromolecular formulation, 8mg/kg of Doxil, which is an adriamycin (hereinafter, ADR)-encapsulating type liposome formulation, and 8mg/kg of PEGylated adriamycin containing a low pH responsive cleavable bond (hereinafter, referred to Micelle-ADR) (refer to Bae, Y. et al., Bioconjugate Chem. 16, 122-130 (2005)) were administered via the tail vein 24 hours before the animals were sacrificed. In addition, 1mg/kg of LY or control was simultaneously administered once intraperitoneally. Animals were sacrificed to extract the tumor mass, (i) frozen samples were prepared, and in addition, (ii) the total amount of ADR within the tissue fragment was quantified by the HPLC method. For the frozen sections by (i), utilizing the auto-fluorescence of ADR, the tissue distribution of ADR was observed using a confocal microscope. Note that, in so doing, it was kept in mind that, while ADR encapsulated by a liposome has auto-fluorescence, ADR bonded to PEG has no auto-fluorescence due to the shielding effect of PEG, and only ADR captured and dissociated in an intracellular low pH environment has auto-fluorescence.

### Result:

(i) When the tissue distribution was observed by auto-fluorescence (in Fig. 6-1, T is tumor cell mass, other sites are cancer stroma), Doxil distribution was found to be mainly in the stromal potion of the vascular periphery 24 hours after administration. In the presence of TGF-β inhibitor, the distribution was over a wider range (refer to Fig. 6-1). (ii) The relative distribution amount, by the HPLC method, was 2.18±0.40 in the presence of TGF- β inhibitor with respect to 1.00±0.15 for the control (p=0.015) (refer to Fig. 6-2).

Next, in the condition of combined application of Micelle-ADR and LY, (i) the release of ADR in cancer cells increased remarkably, and a thorough distribution within the tumor cell group was observed. In the condition of administration of Micelle-ADR alone, ADR was released only in stroma cells and the outermost edges of the tumor mass. This was thought to be caused by the Micelle-ADR being distributed mainly in the stroma and not having reached the tumor cells (Fig. 6-1 refer to). (ii) The total amount of ADR, by the HPLC method, was also shown to be significantly higher in the LY+Micelle-ADR group compared to the lone administration group (P=0.004) (refer to Fig. 6-2).

### 3-6. Smad4-deficient pancreatic adenocarcinoma cell (BxPC3) - Doxil - Micelle-ADR - TGF- β inhibitor growth curve

### Method:

Four weeks-old male nude mice were subcutaneously injected in the abdominal wall with 5×10⁶ counts of Smad4-deficient pancreatic adenocarcinoma cells (BxPC3), and when approximately two weeks have elapsed from the transplantation, those in which the cancer cell mass entered a proliferative phase were used in the experiment as xenograft models of pancreatic adenocarcinoma. For each of 8mg/kg of Doxil, 8mg/kg of Micelle-ADR or 8mg/kg of ordinary ADR as anticancer agent, or no anticancer agent, which was administrated via the tail vein, administration was carried out only once. Simultaneously to anticancer agent administration, intraperitoneal administration was carried out only once, with 1mg/kg of LY364947 (hereinafter, LY; Inhib in the figures) as TGF- β inhibitor or a control. Experiment was carried out for eight conditions combining the above 4 × 2 [modes of administration], with n=5 each. For these animals, the approximated values of tumor volume were observed continuously for 14 days. The tumor volumes were approximated by long side of tumor mass × (short side)² and compared, with 1 as the volume at the administration start time for each tumor mass.

### Result:

On the last day, [the values] were 4.77±0.50 for the control group, 4.83±0.54 for the TGF- β inhibitor alone group, 4.70±0.32 for the ADR alone group, 4.24±0.12 for the TGF- β inhibitor and ADR combined application group, 3.96±0.43 for the Doxil alone group, 2.79±0.28 for the TGF- β inhibitor and Doxil combined application group, 4.16±0.29 for the macromolecular micelle alone group and 2.08±0.20 for the TGF- β inhibitor and macromolecular micelle combined application group. Among these, only two groups are groups with a significant difference by a test according to the MANOVA method, i.e., TGF- β inhibitor/Doxil combined application group (p=0.017 with respect to the control group) and the TGF- β inhibitor and macromolecular micelle combined application group (p=0.0003 with respect to the control group); no significant effect was observed for both Doxil and macromolecular micelle with alone administration. The evolution is shown in the graph of Fig. 7.

### 3-7. Smad4-deficient pancreatic adenocarcinoma cell (BxPC3) - macromolecular micelle-LY growth curve

### Method:

Four weeks-old male nude mice were subcutaneously injected in the abdominal wall with 5×10⁶ counts of Smad4-deficient pancreatic adenocarcinoma cells (BxPC3), and when approximately two weeks have elapsed from the transplantation, those in which the cancer cell mass entered a proliferative phase were used in the experiment as xenograft models of pancreatic adenocarcinoma. For each of, 16mg/kg of Micelle-ADR or 8mg/kg of ADR as anticancer agent, or no anticancer agent, administration via the tail vein was carried out three times spaced by four days. As TGF-β inhibitor, 1mg/kg of LY364947 (hereinafter, referred to as LY) or control was administered three times a week intraperitoneally. The experiment was carried out for the six conditions combining the above 3×2 types [of administration modes], with n=5 each. For these animals, the approximated values of tumor volume were observed continuously for 16 days. For the tumor volumes, those that have been approximated by long side of tumor mass × (short side)² were compared, taking 1 as the value at the administration start time for each tumor mass. In addition, on the last day, the animals were sacrificed to extract the tumor mass, and a histological search was carried out.

### Result:

On the last day, [the values] were 4.69±0.46 for the control group, 4.34±0.37 for the LY alone group, 3.49±0.20 for the ADR alone group, 3.05±0.47 for the Micelle-ADR alone group, 3.49±0.32 for the LY+ADR combined application group and 1.06±0.13 for the LY+Micelle-ADR combined application group (here, the test of difference for the LY+Micelle-ADR combined application group with respect to the Micelle-ADR alone group: P=0.0005). The evolution is shown in the graph of Fig. 8.

### 3-8. MIA PaCa-2 - micelle-LY, tumor growth curve

### Method:

Treatments similar to 3-7 were carried out except that a TGFbRII-mutant pancreatic adenocarcinoma cell line MIA PaCa-2 (ATCC No. CRL-1420) was used.

### Result:

On the last day (16th day), [the values] were 2.91±0.30 for the control group, 2.75±0.08 for the LY alone administration group, 0.87±0.19 for the ADR alone administration group, 0.91±0.10 for the Micelle-ADR alone administration group, 1.45±0.11 for the LY+ADR combined application group and 0.46±0.14 for the LY+Micelle-ADR combined application group (here, the test of the difference for the LY+Micelle-ADR combined application group: p=0.0003). The evolution is shown in the graph of Fig. 9.

### 3-9. MIA PaCa-2 - micelle-LY, intratumoral drug dynamics Method:

Treatments similar to 3-2 were carried out, except that a TGFbRII-mutant pancreatic adenocarcinoma cell line MIA PaCa-2 was used. Only the tumor mass was searched.

### Result:

By observation of ADR auto-fluorescence, incorporation of ADR into tumor cell was observed to increase only in the case of LY+Micelle-ADR combined application (refer to Fig. 10-1). This strongly suggests that influence of TGF-β signaling inhibitor on angiogenesis is independent from the type of cancer and fixed. In addition, as a result of quantification by the HPLC method, the total amount of ADR accumulated in the LY+Micelle-ADR combined application group was shown to be significantly higher compared to the other groups (p=0.0007) (refer to Fig. 10-2).

### 3-10. MIA PaCa-2 liver metastatic foci - micelle-LY, intratumoral drug dynamics

### Method:

A subcutaneous tumor was first generated in nude mouse using a TGFbRII-mutant pancreatic adenocarcinoma cell line MIA PaCa-2, this was further transplanted into the pancreas of another nude mouse and this was used when 4 weeks have elapsed. For the remainder, treatments similar to 3-7 were carried out. Only tumor mass and surrounding hepatic tissue were searched.

### Result:

By observation of ADR auto-fluorescence, incorporation of ADR into metastasizing tumor cell mass was observed to increase only in the case of LY+Micelle-ADR combined application (refer to Fig. 11).

### 3-11. OCUM-2MLN lymph node metastatic foci-micelle-LY, anti-metastatic effect

### Method:

Scirrhous gastric cancer cell line OCUM-2MLN was injected into the gastric wall of a nude mouse and from two weeks later was used as a scirrhous gastric cancer orthotopic graft model. For each of 16mg/kg of Micelle-ADR as anticancer agent or no anticancer agent, administration via the tail vein was carried out three times spaced by four days. Intraperitoneal administration was carried out three times a week with 1mg/kg of LY as TGF- β inhibitor or a control. On the 16th day the animal was sacrificed to measure the total weight of the involved lymph node.

### Result:

The total weight of involved lymph node was suppressed most in the TGF-β inhibitor and micelle combined application-administered group (refer to Fig. 12).

### 3-12. BxPC3-micelle-TbRII-Fc, intratumoral drug dynamics

### Method:

In an identical system to 3-1 described above, TbRII-Fc (R&D) was used as TGF- β inhibitor for intraperitoneal administration. For the dose, 2.5mg/kg was used, which was reported not to inhibit fibrosis in Non-patent Reference 12 (was set with the thought that the amount was the same, since an inhibition of fibrosis was not observed significantly with the LY dose used this time). The anticancer agent applied in combination was 8mg/kg of Micelle-ADR only. Only tumor mass was searched.

### Result:

Similar effects to those observed by observation of ADR auto-fluorescence for the LY combined application in 3-2 were observed in both the auto-fluorescence observation method and the HPLC method (refer to Fig. 13-1 (a), (b) and Fig. 13-2 (a)).

### 3-13. BxPC3 - micelle-anti-VEGF antibody, intratumoral drug dynamics

### Method:

In an identical system to 3-1, 2.5mg/kg of anti-VEGF antibody (R&D) was used in intraperitoneal administration as an agent that also acts on neovasculature but having a completely different type of mechanism of action to TGF- β signaling inhibitor, inhibiting angiogenesis by causing neovasculature to regress or normalize. The anticancer agent applied in combination was 8mg/kg of Micelle-ADR only. Only tumor mass was searched.

### Result:

By observation of ADR auto-fluorescence, in contrast to the case of TGF- β signaling inhibitor combined application, when anti-VEGF antibody was applied in combination, incorporation into tumor cell was almost not observed in the combined application group for the same antibody and the control group. In addition, when quantified by the HPLC method, no significant difference was observed between the anti-VEGF antibody combined application group and the control group. This suggests that the mechanism of facilitation of neovasculature leakiness by inhibition of TGF- β signaling, in particular when adopted in a macromolecular antitumor agent, is clearly preferable compared to application in combination with the mechanism of regression or normalization of blood vessels, which is an existing angiogenesis inhibition method (refer to Fig. 13-1 (C) and (D), and Fig. 13-2 (b)).

### 3-14. Micelle-LY, drug in vivo dynamics

Each of 8mg/kg of Micelle-ADR or 8mg/kg of ADR as anticancer agent was administered to tumor-bearing nude mice via the tail vein. As TGF- β inhibitor, 1mg/kg of LY or a control was administered intraperitoneally. Experiment was carried out for the four conditions combining the above2 × 2 types [of administration modes], with n=3. In the administration, four hours later, these animals were sacrificed, blood, heart, liver, spleen and kidney were extracted, (i) frozen samples were prepared, and in addition, (ii) the total amount of ADR contained in the tissue fragment was quantified by the HPLC method. In addition, the surrounding of the tail vein where the agents were administered was observation.

### Result:

From (ii), in the case of Micelle-ADR administration, in all organs measured, no significant difference of P=0.05 or less was observed between LY administration and non-administration. Also according to (i) no clear difference in the organ distribution between LY administration and non-administration was observed. Consequently, it was supposed that, in normal organs, there is almost no deterioration of adverse effects of an anticancer agent due to LY administration. In addition, no deterioration of anticancer agent-related phlebitis due to combined administration of LY was observed (refer to Fig. 14-1 and Fig. 14-2).

### Industrial Applicability

According to the present invention, based on the discovery of a novel effect of a TGF-β signaling inhibitor, which is the increase in leakiness without causing neovasculature to regress in tumor tissue, a novel medical application of the inhibitor, as well as, a system in which, by the combined use of the inhibitor and an antitumor active substance or an imaging agent, antitumoral activity is increased or the target access selectivity of the imaging agent is increased, are provided. Therefore, the present invention can be used in a wide range of related industries, and the pharmaceutical industry to begin with.

## Claims

1. A medicinal formulation comprising a Transforming Growth Factor β (TGF- β) signaling inhibitor as an active ingredient and a pharmaceutically acceptable excipient, for increasing leakiness without causing neovasculature to regress in a tumor tissue.

2. The medicinal formulation according to Claim 1, wherein the TGF-β signaling inhibitor further causes a decrease in intratumoral fibrous components.

3. The medicinal formulation according to Claim 1, wherein the capability to deliver a drug to cancer cell is enhanced by increasing leakiness without causing neovasculature to regress in the tumor tissue, and depending on the circumstances, decreasing intratumoral fibrous components.

4. The medicinal formulation according to Claim 1, wherein the TGF- β signaling inhibitor is selected from the group consisting of a soluble TGF- β Type I receptor, a soluble TGF- β Type II receptor, a soluble TGF- β Type III receptor, an anti-TGF- β antibody, an anti-TGF- β Type I receptor antibody, an anti-TGF- β Type II receptor antibody, an anti-TGF- β Type III receptor antibody and a TGF- β antisense oligonucleotide or siRNA.

5. The medicinal formulation according to Claim 1, wherein the TGF-β signaling inhibitor is selected from the group consisting of a TGF-β Type I receptor kinase inhibitor and a TGF- β Type II receptor kinase inhibitor.

6. The medicinal formulation according to Claim 5, wherein the TGF-β Type I receptor kinase inhibitor and the TGF- β Type II receptor kinase inhibitor are low molecular weight compounds selected from the group consisting of a dihydropyrrolopyrazole-based scaffold, an imidazole-based scaffold, a pyrazolopyridine-based scaffold, a pyrazole-based scaffold, an imidazopyridine-based scaffold, a triazole-based scaffold, a pyridopyrimidine-based scaffold, a pyrrolopyrazole-based scaffold and an isothiazole-based scaffold.

7. The medicinal formulation according to Claim 1, wherein the tumor is selected from a tumor group considered to be resistant to chemotherapy due to stromal components being abundant or of a poorly neovascularized tissue type, or selected from the group consisting of an intractable digestive organ tumor, an intractable soft tissue tumor and a breast cancer with advanced fibrosis.

8. A combination for tumor treatment, wherein a TGF- β signaling inhibitor as an active ingredient and an antitumor active substance as an active ingredient have been combined.

9. A combination for tumor treatment, in which a TGF-β signaling inhibitor as an active ingredient and an antitumor active substance as an active ingredient have been combined, wherein the antitumor active substance has been modified so as to improve delivery capability to a tumor cell or a tumor tissue.

10. The combination according to Claim 9, wherein the modified antitumor active substance is in the form of a conjugate of a high molecular weight carrier and an antitumor active substance, or the antitumor active substance is in an encapsulated form by a liposome or a dendrimer or a macromolecular micelle.

11. The combination according to Claim 9, wherein the modified antitumor active substance is a conjugate of a high molecular weight carrier and an antitumor active substance, and is selected from the group consisting of a complex of a polymer or a copolymer holding a cationic charged polymer or a cationic charged polymer segment and an oligo- or polynucleotide *per se* or a derivative thereof, and a conjugate of an antibody and an antitumor active substance bonded through a hydrazone bond or a disulfide bond cleavable *in vivo.*

12. The combination according to Claim 8, wherein the antitumor active substance is selected from the group consisting of an oligo- or polynucleotide, and derivative thereof, functioning to inhibit the expression of an oncogene or to express a cancer suppressor gene, an anti-metabolite, an alkylating drug, a platinum formulation, an antibiotic, a plant constituent extract, and a radioactive nuclide or substance.

13. The combination according to Claim 9, wherein the antitumor active substance is selected from the group consisting of an oligo- or polynucleotide, and derivative thereof, functioning to inhibit the expression of an oncogene or to express a cancer suppressor gene, an anti-metabolite, an alkylating drug, a platinum formulation, an antibiotic, a plant constituent extract, and a radioactive nuclide or substance.

14. The combination according to Claim 10, wherein the liposome is formed from polycationic lipids, and the macromolecular micelle is formed from a carrier selected from the group consisting of a block copolymer comprising a hydrophilic polymer chain segment and a hydrophobic polymer chain segment, and a block copolymer comprising a hydrophilic polymer chain segment and a charged polymer segment.

15. The combination according to Claim 14, wherein the hydrophilic polymer chain segment is derived from poly(ethylene glycol), and the hydrophobic polymer chain segment is derived from a polymer selected from the group consisting of poly(hydrophobic amino acid), poly(aspartic acid ester) and poly(glutamic acid ester), poly(lactide), poly(lactone) and copolymers thereof, and the charged polymer segment is derived from a polymer selected from the group consisting of poly(glutamic acid), poly(aspartic acid), poly(lysine), poly(N,N-dialkylaminoalkyl(meta)acrylate) and poly(ethyleneimine).

16. The combination according to Claim 8, wherein the TGF- β signaling inhibitor is selected from the group consisting of a soluble TGF- β Type I receptor, a soluble TGF-β Type II receptor, a soluble TGF- β Type III receptor, an anti-TGF-β antibody, an anti-TGF- β Type I receptor antibody, an anti-TGF-β Type II receptor antibody, an anti-TGF- β Type III receptor antibody and a TGF- β antisense oligonucleotide.

17. The combination according to Claim 9, wherein the TGF- β signaling inhibitor is selected from the group consisting of a soluble TGF- β Type I receptor, a soluble TGF-β Type II receptor, a soluble TGF- β Type III receptor, an anti-TGF-β antibody, an anti-TGF- β Type I receptor antibody, an anti-TGF-β Type II receptor antibody, an anti-TGF- β Type III receptor antibody and a TGF- β antisense oligonucleotide.

18. The combination according to Claim 8, wherein the TGF- β signaling inhibitor is selected from the group consisting of a TGF- β Type I receptor kinase inhibitor and a TGF- β Type II receptor kinase inhibitor.

19. The combination according to Claim 9, wherein the TGF- β signaling inhibitor is selected from the group consisting of a TGF- β Type I receptor kinase inhibitor and a TGF- β Type II receptor kinase inhibitor.

20. The combination according to Claim 18, wherein the TGF-β Type I receptor kinase inhibitor and the TGF- β Type II receptor kinase inhibitor are low molecular weight compounds selected from the group consisting of a dihydropyrrolopyrazole-based scaffold, an imidazole-based scaffold, a pyrazolopyridine-based scaffold, a pyrazole-based scaffold, an imidazopyridine-based scaffold, a triazole-based scaffold, a pyridopyrimidine-based scaffold, a pyrrolopyrazole-based scaffold and an isothiazole-based scaffold.

21. The combination according to Claim 19, wherein the TGF-β Type I receptor kinase inhibitor and the TGF- β Type II receptor kinase inhibitor are low molecular weight compounds selected from the group consisting of a dihydropyrrolopyrazole-based scaffold, an imidazole-based scaffold, a pyrazolopyridine-based scaffold, a pyrazole-based scaffold, an imidazopyridine-based scaffold, a triazole-based scaffold, a pyridopyrimidine-based scaffold, a pyrrolopyrazole-based scaffold and an isothiazole-based scaffold.

22. The combination according to Claim 8, wherein the tumor to be treated is selected from a tumor group considered to be resistant to chemotherapy due to stromal components being abundant or of a poorly neovascularized tissue type.

23. The combination according to Claim 9, wherein the tumor to be treated is selected from a tumor group considered to be resistant to chemotherapy due to stromal components being abundant or of a poorly neovascularized tissue type.

24. The combination according to Claim 8, wherein the tumor to be treated is selected from the group consisting of an intractable digestive organ tumor, an intractable soft tissue tumor and a breast cancer with advanced fibrosis.

25. The combination according to Claim 9, wherein the tumor to be treated is selected from the group consisting of an intractable digestive organ tumor, an intractable soft tissue tumor and a breast cancer with advanced fibrosis.

26. A kit for tumor treatment, comprising a medicinal formulation containing a TGF- β signaling inhibitor as an active ingredient and a medicinal formulation containing an antitumor active substance as an active ingredient.

27. A kit for tumor treatment, comprising a medicinal formulation containing a TGF-β signaling inhibitor as an active ingredient and a medicinal formulation containing an antitumor active substance as an active ingredient, wherein the antitumor active substance has been modified so as to improve delivery capability to a tumor cell or a tumor tissue.

28. The kit according to Claim 26, wherein the medicinal formulation containing the TGF- β signaling inhibitor is selected from the group consisting of an agent for venous injection use, an agent for intratumoral injection use and an agent for subcutaneous injection use, and the medicinal formulation containing the antitumor active substance is selected from the group consisting of an agent for venous injection use, an agent for intratumoral injection use, an agent for subcutaneous injection use, an agent for peroral use and an agent for rectal injection use.

29. The kit according to Claim 27, wherein the medicinal formulation containing the TGF- β signaling inhibitor is selected from the group consisting of an agent for venous injection use, an agent for intratumoral injection use and an agent for subcutaneous injection use, and medicinal formulation containing the modified antitumor active substance is selected from the group consisting of an agent for venous injection use, an agent for intratumoral injection use, an agent for subcutaneous injection use, an agent for peroral use and an agent for rectal injection use.

30. The kit according to Claim 26, wherein the TGF- β signaling inhibitor is selected from the group consisting of a soluble TGF- β Type I receptor, a soluble TGF- β Type II receptor, a soluble TGF- β Type III receptor, an anti-TGF- β antibody, an anti-TGF- β Type I receptor antibody, an anti-TGF-β Type II receptor antibody, an anti-TGF-β Type III receptor antibody, a TGF- β antisense oligonucleotide, a TGF- β Type I receptor kinase inhibitor and a TGF- β Type II receptor kinase inhibitor.

31. The kit according to Claim 27, wherein TGF- β signaling inhibitor is selected from the group consisting of a soluble TGF- β Type I receptor, a soluble TGF- β Type II receptor, a soluble TGF- β Type III receptor, an anti-TGF- β antibody, an anti-TGF- β Type I receptor antibody, an anti-TGF- β Type II receptor antibody, an anti-TGF- β Type III receptor antibody, a TGF- β antisense oligonucleotide, a TGF- β Type I receptor kinase inhibitor and a TGF- β Type II receptor kinase inhibitor.

32. The kit according to Claim 30, wherein the TGF- β Type I receptor kinase inhibitor and the TGF-β Type II receptor kinase inhibitor are low molecular weight compounds selected from the group consisting of a dihydropyrrolopyrazole-based scaffold, an imidazole-based scaffold, a pyrazolopyridine-based scaffold, a pyrazole-based scaffold, an imidazopyridine-based scaffold, a triazole-based scaffold, a pyridopyrimidine-based scaffold, a pyrrolopyrazole-based scaffold and an isothiazole-based scaffold.

33. The kit according to Claim 31, wherein the TGF- β Type I receptor kinase inhibitor and the TGF-β Type II receptor kinase inhibitor are low molecular weight compounds selected from the group consisting of a dihydropyrrolopyrazole-based scaffold, an imidazole-based scaffold, a pyrazolopyridine-based scaffold, a pyrazole-based scaffold, an imidazopyridine-based scaffold, a triazole-based scaffold, a pyridopyrimidine-based scaffold, a pyrrolopyrazole-based scaffold and an isothiazole-based scaffold.

34. A combination for imaging, wherein a TGF- β signaling inhibitor as an active ingredient and an imaging agent as an active ingredient have been combined.

35. The combination according to Claim 34, wherein the imaging agent has been modified so as to improve delivery capability to a tumor cell or a tumor tissue.

36. The combination according to Claim 35, wherein the modified imaging agent is in the form of a conjugate of a high molecular weight carrier and an antitumor active substance, or the imaging agent is in an encapsulated form by a liposome or a dendrimer or a macromolecular micelle.

37. A method for increasing leakiness without causing neovasculature to regress in the tumor tissue of an individual (subject), comprising administering a therapeutic effective dose of Transforming Growth Factor β (TGF-β) signaling inhibitor to an individual requiring the increase in leakiness without causing the neovasculature to regress.

38. A method for treating a tumor in an individual, comprising administering a TGF- β signaling inhibitor and an antitumor active substance to an individual requiring said treatment, simultaneously or at respectively different times, before and after.

39. A method for treating a tumor in an individual, comprising administering a TGF- β signaling inhibitor and an antitumor active substance modified so as to improve delivery capability to a tumor cell or a tumor tissue, to an individual requiring said treatment, simultaneously or at respectively different times, before and after.

40. A method for imaging a tumor tissue in an individual, comprising administering a TGF-β signaling inhibitor and an imaging agent modified so as to improve delivery capability to a tumor cell or a tumor tissue, to an individual requiring said imaging, simultaneously or at respectively different times, before and after.

41. A use of a Transforming Growth Factor β (TGF-β) signaling inhibitor when preparing a medicinal formulation for increasing leakiness without causing neovasculature to regress in a tumor tissue of an individual (subject).

42. A use of a combination of a TGF- β signaling inhibitor and an antitumor active substance when preparing a medicinal formulation for use in the treatment of a tumor in an individual.

43. A use of a combination of a TGF- β signaling inhibitor and an antitumor active substance modified so as to improve delivery capability to a tumor cell or a tumor tissue, when preparing a medicinal formulation for use in the treatment of a tumor in an individual.

44. A use of a combination of a TGF- β signaling inhibitor and an imaging agent when preparing an imaging composition for use in imaging a tumor tissue of an individual.

45. A use of a combination of a TGF- β signaling inhibitor and an imaging agent modified so as to improve delivery capability to a tumor cell or a tumor tissue, when preparing an imaging composition for use in imaging a tumor tissue of an individual.
